(19) Europäisches Patentamt

European Patent Office

Office européen des brevets

(11) **EP 1 326 708 B1**

(12) **EUROPÄISCHE PATENTSCHRIFT**

(45) Veröffentlichungstag und Bekanntmachung des Hinweises auf die Patenterteilung:
**05.07.2006 Patentblatt 2006/27**

(51) Int Cl.:
**B01J 20/32** (2006.01)

(21) Anmeldenummer: **01984630.2**

(22) Anmeldetag: **14.09.2001**

(86) Internationale Anmeldenummer:
**PCT/EP2001/010678**

(87) Internationale Veröffentlichungsnummer:
**WO 2002/022253 (21.03.2002 Gazette 2002/12)**

(54) **ADSORBENS MIT UNTERSCHIEDLICH MODIFIZIERTEN OBERFLÄCHENBEREICHEN, VERFAHREN ZU DESSEN HERSTELLUNG UND VERWENDUNG DAVON**

ADSORBENT HAVING DIFFERENTLY MODIFIED SURFACE AREAS, METHOD FOR THE PRODUCTION THEREOF AND USE OF THE SAME

ADSORBANT COMPORTANT DES ZONES SUPERFICIELLES A MODIFICATION DIFFERENCIEE, SON PROCEDE DE PRODUCTION ET SON UTILISATION

(84) Benannte Vertragsstaaten:
**AT BE CH CY DE DK ES FI FR GB GR IE IT LI LU MC NL PT SE TR**

(30) Priorität: **14.09.2000 DE 10045434**

(43) Veröffentlichungstag der Anmeldung:
**16.07.2003 Patentblatt 2003/29**

(73) Patentinhaber: **Fresenius Medical Care Deutschland GmbH**
**61352 Bad Homburg (DE)**

(72) Erfinder:
• **BAUMANN, Hanno**
**52076 Aachen (DE)**
• **GERNER, Franz-Josef**
**66606 St. Wendel (DE)**
• **HOFFMANN, Michael**
**52249 Eschweiler (DE)**
• **HORRES, Roland**
**52223 Stolberg (DE)**
• **KOKOTT, Andreas**
**95138 Bad Steben (DE)**
• **LEINENBACH, Hans-Peter**
**66636 Tholey (DE)**
• **MATHAR, Günter**
**52224 Stolberg (DE)**
• **METZGER, Wolfgang**
**66113 Saarbrücken (DE)**

• **OTTO, Veit**
**66606 St. Wendel (DE)**
• **RÜGER, Walter**
**36277 Schenklengsfeld (DE)**
• **SCHIMMEL, Martin**
**66606 St. Wendel (DE)**

(74) Vertreter: **Müller-Boré & Partner Patentanwälte**
**Grafinger Strasse 2**
**81671 München (DE)**

(56) Entgegenhaltungen:
**EP-A- 0 106 769      EP-A- 0 295 809**
**DE-A- 19 856 387      FR-A- 2 653 034**
**GB-A- 2 113 226      US-A- 4 544 485**
**US-A- 5 773 384**

• **DATABASE WPI Section Ch, Week 199350 Derwent Publications Ltd., London, GB; Class A89, AN 1993-400805 XP002208709 & JP 05 302917 A (SEKISUI CHEM IND CO LTD) , 16. November 1993 (1993-11-16)**
• **DATABASE WPI Section Ch, Week 198809 Derwent Publications Ltd., London, GB; Class A13, AN 1988-061318 XP002208708 & JP 63 017906 A (TOKYO ORGANIC CHEM IND CO LTD), 25. Januar 1988 (1988-01-25)**

## EP 1 326 708 B1

**Beschreibung**

Die vorliegende Erfindung betrifft ein Adsorbens für Vollblut in Form von im wesentlichen sphärischen, nicht aggregierten Teilchen, welches ein poröses organisches Trägermaterial mit einer mittleren Porengröße von $\leq 1{,}5\ \mu m$ umfaßt, wobei maximal 50 % des Porenvolumens in Poren vorliegen kann, welche eine Porengröße von $> 1{,}5\ \mu m$ aufweisen, wobei die äußeren Oberflächen des porösen Trägermaterials mindestens eine Oberflächenmodifizierung $M_1$ aufweisen, wodurch die äußere Oberfläche im wesentlichen nicht mit Blutzellen wechselwirkt, und die inneren Oberflächen des porösen Trägermaterials, d.h. die Oberflächen der Poren des porösen Trägermaterials, mindestens eine Oberflächenmodifizierung $M_2$ aufweisen, welche mit in Blut enthaltenen Substanzen wechselwirkt. Ferner betrifft die vorliegende Erfindung ein Verfahren zur Herstellung eines derartigen Adsorbens, die Verwendung dieses Adsorbens, sowie einen Adsorber, welcher das erfindungsgemäße Adsorbens in einem Gehäuse umfaßt.

Adsorbentien sind in der Medizintechnik weit verbreitet. Häufig beschrieben werden Adsorber mit Adsorbentien, die aus Blut oder Blutbestandteilen Lipoproteine mit niedriger Dichte (LDL) entfernen bzw. deren Konzentration herabsetzen, wie dies aus der DE 39 32 971 bekannt ist. Diese Druckschrift beschreibt das Adsorbensmaterial als einen organischen Träger mit festgelegter Partikelgröße und Ausschlußgrenze, welcher auf seiner Oberfläche eine Funktionalisierung trägt, an den das LDL-Molekül bindet.

Es gibt LDL-Adsorber aus porösen Polymethacrylatteilchen, welche mit Polyacrylsäure (PAS) beschichtet sind, wobei PAS sowohl auf der äußeren, die Teilchen begrenzenden Oberfläche als auch auf der inneren, die Poren umschließenden Oberfläche gebunden vorliegt. Da die Blutzellen nur geringe Wechselwirkungen mit PAS zeigen, ist ein derartiges Adsorbens vollbluttauglich, d.h. die Adsorbensteilchen lassen die Blutzellen passieren, ohne mit ihnen wechselzuwirken, d.h. ohne sie zu aktivieren, zu binden oder zu schädigen. Somit kann gemäß diesem Verfahren das vollständige Blut ohne vorherige Abtrennung der Blutzellen über den Adsorber geleitet werden. Die Abtrennung des LDL erfolgt hier über einen Größenausschlußmechanismus und nicht über eine spezifische Bindung des LDL an eine auf dem Träger haftende, funktionelle Gruppe.

Die Vollblutverträglichkeit eines Adsorbens ist sehr ungewöhnlich, da das Trägermaterial normalerweise zur Komplementaktivierung führt und/oder eine Thrombozytenaggregation und -adhäsion auslöst.

Die Vollblutverträglichkeit ist insbesondere von der verwendeten Beschichtung mit funktionellen Gruppen abhängig. Bei Verwendung bestimmter, im Stand der Technik bekannter funktioneller Gruppen ist es nicht möglich, vollblutverträgliche Adsorber herzustellen, weil sie mit den Blutzellen wechselwirken. Ein Beispiel dafür ist ein Adsorber zur Bindung von Immunkomplexen, welcher auf seiner Oberfläche das Protein C1q als funktionelle Gruppe trägt. Das Protein geht mit Immunglobulinen eine Wechselwirkung ein und kann diese so aus ihren Lösungen entfernen. Eine Bindungsstelle zum Protein C1q weisen jedoch auch Blutzellen, insbesondere Thrombozyten, auf, und sie werden ebenfalls an solche Adsorbentien gebunden. Vollblut kann diese Adsorbentien somit nicht unbeeinträchtigt passieren. Die gleichen Probleme treten auch bei sogenannten Fibrinogenadsorbern auf.

In derartigen Fällen müssen die Blutzellen zunächst vom Blutplasma abgetrennt werden, woraufhin nur das Plasma über den Adsorber geleitet wird. Nach der Abtrennung der Immunkomplexe durch die Bindung an das Protein C1q müssen die Blutzellen wieder mit dem gereinigten Blutplasma vereint werden. Diese Prozedur ist aufwendig und für den Patienten belastend und risikoreich.

Die in der Literatur (beispielsweise bei Dräger et al., Eur. J. Clin. Invest, 1998, 28 (12); US-A-5 476 715) beschriebenen Vollblutadsorbentien bestehen aus Teilchen, welche so groß sind, daß sie Zwischenräume bilden, in denen sich die Blutzellen bewegen können. Ferner weisen die Teilchen Poren auf, welche zu einer inneren Oberfläche führen. Diese Poren weisen eine ausreichende Größe auf, daß sogar Makromoleküle in sie eindringen können. Die Poren sind jedoch so klein gewählt, daß das Eindringen von Blutzellen ausgeschlossen ist. Die Blutzellen haben somit nur Kontakt zur äußeren Oberfläche der Teilchen. Ferner müssen diese Teilchen gemäß EP 0 424 698 möglichst sphärisch und unaggregiert sein, um eine "glatte" sowie auch eine inerte Außenseite aufzuweisen, so daß die Thrombozyten daran abgleiten.

DE 198 42 785 A1 beschreibt poröse Materialien, deren Oberflächen chemisch so funktionalisiert sind, daß die äußere Oberfläche der porösen Materialien elektroneutral und hydrophil ist, während die innere Oberfläche mit sogenannten funktionellen Liganden ausgestaltet sein kann. Vollblutverträgliche Materialien werden jedoch nicht beschrieben. Diese porösen Materialien werden hergestellt, indem zunächst Epoxidgruppen in einen porösen Basisträger eingeführt werden, wobei sowohl die Porenoberflächen als auch die Außenflächen des Basisträgers mit Epoxidgruppen funktionalisiert werden und anschließend die Epoxidgruppen katalytisch durch Reaktion mit einem Nuldeophil geöffnet werden. Dabei kommt ein partikulärer Katalysator mit einer Partikelgröße größer als der mittlere Porendurchmesser des porösen Basisträgers zur Anwendung, wodurch eine Umsetzung in den Poren nicht stattfinden kann. Abschließend werden die verbliebenen Epoxidgruppen an den Porenoberflächen durch Einführen von funktionellen Liganden umgesetzt. Die Verwendung eines partikulären Katalysators bringt jedoch erhebliche Nachteile mit sich, da die Abtrennung des teilchenförmigen Katalysators vom beschichteten Trägermaterial schwierig ist. Auch darf und kann eine Reaktion nur an Kontaktstellen zwischen den Katalysatorteilchen und den Trägermaterialteilchen stattfinden, wodurch sehr lange Reaktionszeiten und extensives Rühren erforderlich sind, um eine annähernd vollständige Umsetzung der Ober fläche zu erreichen. Das lange Rühren kann ferner zu einem Abrieb der Katalysatorteilchen führen, wodurch der Adsorber mit Katalysatorresten verunreinigt werden kann. Ferner kann auch, wenn magnetische Katalysatorteilchen verwendet werden, nicht ausgeschlossen werden, daß Katalysatorteilchen im Adsorbens zurückbleiben. Diese Katalysatorteilchen können jedoch insbesondere bei der Reinigung von Vollblut durch Wechselwirkungen mit Blutbestandteilen zu Problemen führen, so daß ein derartiger Katalysator nicht vollbluttauglich ist.

EP 0 295 809 A2 betrifft ein Adsorbens (Säulenfüllmaterial) zur flüssigchromatographischen Analyse von Blutserum. Der Träger für dieses Adsorbens ist anorganisch.

US-A-4 544 485 betrifft ein Säulenfüllmaterial für die Flüssigchromatographie.

FR-A-2 653 034 betrifft ein Säulenfüllmaterial für die Flüssigchromatographie zur chromatographischen Abtrennung von zum Beispiel Proteinen.

JP-A-5 302 917 betrifft ein Säulenfüllmaterial für die Flüssigchromatographie zur Abtrennung von Proteinen aus biologischem Material.

US-A-5 773 384 betrifft ein Sorbens zur Entfernung von Giftstoffen aus Blut oder Plasma, welches eine Vielzahl übervernetzter Perlen aus einem Harz vom Polystyroltyp aufweist, deren Oberfläche so modifiziert ist, daß die Adsorption von großen Proteinen und Thrombozyten verhindert und die Aktivierung des Blut-Komplementsystems minimiert werden, ohne die Zugänglichkeit des inneren Adsorptionsraumes der Perlen für kleine und mittelgroße Giftstoffmoleküle merklich zu beeinträchtigen.

EP 0 106 769 A1 betrifft einen teilchenförmigen Träger für die Herstellung eines Adsorbens für die Affinitäts-Säulenchromatographie.

JP-A-1 123 145 betrifft ein Säulenfüllmaterial für die Chromatograhpie auf Basis von Silicagel.

JP-A-3 107 759 betrifft ein Säulenfüllmaterial für die Flüssigchromatographie zur Abtrennung und Analyse von zum Beispiel Arzneimitteln oder Giftstoffen aus biologischen Flüssigkeiten, wie beispielsweise Serum.

Somit bestand die Aufgabe der vorliegenden Erfindung darin, ein Adsorbens bereitzustellen, welches völlblutverträglich ist und an für Blutzellen nicht erreichbaren

Stellen Gruppierungen trägt, welche mit aus dem Blut abzutrennenden Substanzen in Wechselwirkung treten können. Ferner soll ein Verfahren bereitgestellt werden, mit welchem ein derartiges Adsorbens einfach und wirtschaftlich hergestellt werden kann.

Diese Aufgabe wird durch die in den Ansprüchen gekennzeichneten Ausführungsformen gelöst.

Insbesondere wird ein Adsorbens für Vollblut in Form von im wesentlichen sphärischen, nicht aggregierten Teilchen bereitgestellt, welches ein poröses organisches Trägermaterial mit einer mittleren Porengröße von $\leq 1,5 \ \mu m$ umfaßt, wobei maximal 50 % des Porenvolumens in Poren vorliegen kann, welche eine Porengröße von $> 1,5 \ \mu m$ aufweisen, wobei die äußeren Oberflächen des porösen Trägermaterials mindestens eine Oberflächenmodifizierung $M_1$ aufweisen, wodurch die äußere Oberfläche im wesentlichen nicht mit Blutzellen wechselwirkt, und die inneren Oberflächen des porösen Trägermaterials, d.h. die Oberflächen der Poren des porösen Trägermaterials, mindestens eine Oberflächenmodifizierung $M_2$ aufweisen, welche mit in Blut enthaltenen Substanzen wechselwirkt.

Die Figuren zeigen:

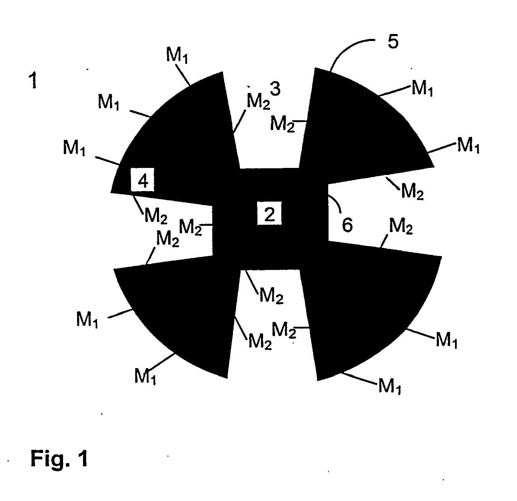

Figur 1 zeigt schematisch einen Querschnitt durch eine Ausführungsform eines erfindungsgemäßen Adsorbensteilchens 1, welches ein Trägermaterial 2 mit Poren 3 und modifizierbare Bindungsstellen 4, sowie damit verbundene Oberflächenmodifizierungen $M_1$ und $M_2$ aufweist. Die Poren 3 des Trägermaterials 2 werden von "inneren" Oberflächen 6 umschlossen, welche Oberfiächenmodifizierungen $M_2$ gebunden aufweisen. Die Bindungsstellen auf der "äußeren" Oberfläche 5, d.h. der das Adsorberteilchen 1 umschließenden Oberfläche, sind mit von den Oberflächenmodifizierungen $M_2$ an den inneren Oberflächen 6 verschiedenen Oberflächenmodifizierungen $M_1$ besetzt.

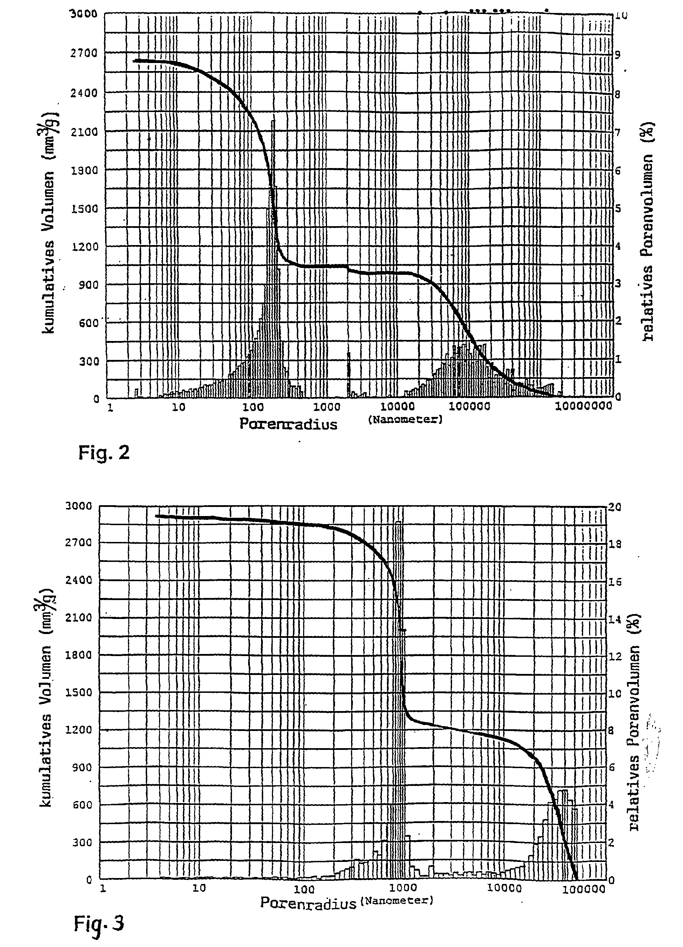

Figuren 2 und 3 zeigen beispielhaft zwei Porengrößenverteilungen, welche gemäß der nachstehend beschriebenen Methode analog DIN 66 133 an einem erfindungsgemäßen Adsorbens gemessen wurden.

Erfindungsgemäß wird unter den "äußeren Oberflächen" eines Teilchens die das Teilchen umschließende Oberfläche verstanden.

Unter den "inneren Oberflächen" eines Teilchens werden die Oberflächen verstanden, welche die Poren des Teilchens umschließen, d.h. die Oberflächen der Poren des Teilchens.

Der Begriff "Wechselwirkung" umfaßt erfindungsgemäß eine Aktivierung, Anbindung, Umsetzung und/oder Schädigung von im Vollblut enthaltenen Substanzen und/oder Zellen. Erfindungsgemäß ist eine selektive Wechselwirkung bevorzugt. Die Wechselwirkung kann gemäß einer weiteren Ausführungsform auch spezifisch sein.

Das erfindungsgemäße Adsorbens ist teilchenförmig. Die Teilchen des erfindungsgemäßen Adsorbens sind im wesentlichen sphärisch und nicht aggregiert. Sphärische Teilchen haben im Gegensatz zu unregelmäßig geformten Teilchen oder unregelmäßig geformten Aggregaten von Teilchen den Vorteil, daß sie Blutteilchen, wie Thrombozyten, nicht unerwünschterweise zurückhalten.

Das poröse organische Trägermaterial des erfindungsgemäßen Adsorbens weist eine mittlere Porengröße bzw. einen mittleren Porendurchmesser mit einem Wert von ≤1,5 $\mu$m, vorzugsweise ≤ 1,0 $\mu$m, auf, wobei maximal 50 % des Porenvolumens in Poren mit einer Porengröße von > 1,5 $\mu$m vorliegen. Die maximale mittlere Porengröße von 1,5 $\mu$m ist durch die Größe der kleinsten Blutzellen bedingt, welche einen Durchmesser von etwa 2 $\mu$m aufweisen. Bei einer, wie gemäß der vorliegenden Erfindung definierten maximalen mittleren Porengröße ist sichergestellt, daß im wesentlichen keine Blutzellen in die Poren eindringen können. Sofern nur sehr kleine oder gelöste Teilchen aus dem Blut abgetrennt werden sollen, ist es bevor zugt, ein poröses Trägermaterial mit einer geringeren mittleren Porengröße zu wählen, beispielsweise 1 $\mu$m, 0,5 $\mu$m oder 0,3 $\mu$m.

Erfindungsgemäß kann die mittlere Porengröße durch Quecksilberintrusion (Quecksilberporosimetrie) analog DIN 66 133 bestimmt werden. Das Verfahren beruht auf der Messung des in einen porösen Feststoff eingepreßten Quecksilbervolumens in Abhängigkeit vom angewandten Druck. Eine nichtbenetzende Flüssigkeit wie Quecksilber dringt nur unter Druck in ein poröses System ein. Der aufzuwendende Druck ist umgekehrt proportional zur lichten Weite der Porenöffnungen. Dabei werden Poren erfaßt, in die bei dem angewendeten Druck Quecksilber eindringen kann. Für zylindrische Poren ist der Zusammenhang zwischen Porenradius $r_p$ und Druck p durch die Washbum-Gleichung gegeben:

$$r_p = -\frac{2 \cdot \sigma}{p} \cos \vartheta$$

wobei $\sigma$ die Oberflächenspannung des Quecksilbers [N/m] und $\vartheta$ der Kontaktwinkel des Quecksilbers auf der Probe, gemessen durch die flüssige Phase ist. In Abweichung zur DIN Norm 66 133 wird für den Kontaktwinkel der feste Wert 141,3° eingesetzt und es werden bei der Bestimmung der mittleren Porengröße die Zwischenkomvolumina nicht mitgerechnet. Figuren 2 und 3 zeigen beispielhaft die Bestimmung des mittleren Porenvolumens zweier poröser Trägermaterialien, welche gemäß der vorliegenden Erfindung verwendet werden können. Die Zwischenkomvolumina sind jeweils durch den rechten Peak, welcher bei Werten über etwa 10 $\mu$m beginnt, repräsentiert. Die Verteilung der offenen Poren zeigt jeweils der linke Peak. Figur 2 zeigt ein Trägermaterial mit einer relativ kleinen mitderen Porengröße von etwa 200 nm. Figur 3 zeigt ein Trägermaterial mit einer größeren mittleren Porengröße von etwa 1 $\mu$m.

Ferner weisen die Adsorbensteilchen vorzugsweise eine Komgröße von 50 bis 500 $\mu$m, mehr bevorzugt von 100 bis 300 $\mu$m, auf. Bei einem Einsatz in einem extrakorporalen Kreislauf mit Vollblut sollten die Teilchen des Adsorbens eine Komgröße von mindestens 50 $\mu$m aufweisen, da die im Vollblut vorhandenen größten Blutkörperchen bzw. Blutzellen einen Durchmesser von 20 $\mu$m besitzen. Somit muß das Sieb, welches das Adsorbens zurückhält, eine Maschenweite von mindestens 25 $\mu$m, vorzugsweise mindestens 40 $\mu$m, haben, damit alle Blutzellen durchgelassen werden. Ferner ist in den mit Adsorbensteilchen einer Größe von 50 $\mu$m und mehr gepackten Säulen der Zwischenraum zwischen den Teilchen für den Durchlaß von Blutzellen ausreichend. Bei der Auswahl des Siebmaterials ist ferner darauf zu achten, daß die Maschenweite stets kleiner ist, als der Durchmesser der kleinsten Adsorberteilchen.

Das erfindungsgemäße Adsorbens umfaßt ein poröses organisches Trägermaterial. Dabei kann man von einem bereits hergestellten, porösen teilchenförmigen Trägermaterial ausgehen, wobei dessen äußere und innere Oberflächen erfindungsgemäß modifiziert werden. Nach dieser Ausführungsform sind Trägermaterialien geeignet, wie beispielsweise, Kohlenhydrate, Sepharose oder organische Trägermaterialien, wie Copolymere von Acrylaten oder Methacrylaten sowie Polyamiden. Vorzugsweise besteht das Trägermaterial aus von (Meth)Acrylsäureestem und/oder -amiden abgeleiteten Copolymeren. Diese weisen vorzugsweise Epoxidgruppen auf. Unter dem Begriff "(Meth)Acryl" sind sowohl die entsprechenden Acryl- als auch Methacrylverbindungen zu verstehen.

Es ist jedoch auch möglich, nicht von bereits hergestellten Teilchen als Trägermaterial auszugehen, sondem poröse Teilchen auf Polymerbasis ausgehend von den entsprechenden Monomeren herzustellen und die äußeren und inneren Oberflächen der derart erhaltenen porösen Polymerteilchen unterschiedlich zu modifizieren.

Das erfindungsgemäß zu verwendende poröse organische Trägermaterial weist an seinen äußeren und inneren Oberflächen modifizierbare Bindungsstellen auf, welche zu Oberflächenmodifizierungen $M_1$ und/oder $M_2$ umgesetzt werden können. Besonders bevorzugt als Bindungsstelle ist ein gespanntes heterocyclisches System als funktionelle Gruppe, welche unter Ringöffnung, insbesondere nukleophile Ringöffnung durch direkte kovalente Bindung weitere funktionelle Gruppen an ein Trägermaterial binden kann und das Material somit als Oberflächenmodifizierung $M_1$ und/ oder $M_2$ derartige funktionelle Gruppen trägt. Das Trägermaterial enthält als Bindungsstellen für derartige funktionelle Gruppen besonders bevorzugt Oxirangruppen bzw. Epoxidgruppen.

Als Trägermaterial ist ein vemetztes statistisches Copolymer bevorzugt, welches durch Polymerisation von Ethylenglykoldi(meth)acrylat und Glycidyl(meth)acrylat und/oder Allyglycidylether erhalten wird.

In der Ausführungsform der vorliegenden Erfindung, bei der man im ersten Schritt ein teilchenförmiges, poröses organisches Trägermaterial bereitstellt, ist ein durch Polymerisation der monomeren Einheiten

(A) (Meth)Acrylamid in einer Menge von 10 bis 30 Gew.-%,

(B) N,N'-Methylen-bis(meth)acrylamid in einer Menge von 30 bis 80 Gew.%, und

(C) Allylglycidylether und/oder Glycidyl(meth)acrylat in einer Menge von 10 bis 20 Gew.-%, jeweils bezogen auf das Gesamtgewicht der monomeren Einheiten,

hergestelltes, vernetztes statistisches Copolymer bevorzugt. Die Netzwerkstruktur wird vorzugsweise durch Suspensionspolymerisation hergestellt. Ein derartiges Copolymer ist im Handel unter der Bezeichnung Eupergit C250L bzw. Eupergit FE162 von Röhm GmbH erhältlich.

Unter einer "Oberflächenmodifizierung° wird gemäß einer Ausführungsform erfindungsgemäß eine vorzugsweise organische Endgruppe, Substanz, Verbindung und/oder Vorläuferverbindung verstanden, welche zum Binden an Bindungsstellen eines Trägermaterials geeignet ist und welche, wenn sie an ein Trägermaterial gebunden ist, die Fähigkeit zur vorzugsweise selektiven Wechselwirkung mit bestimmten, in einer Lösung enthaltenen Substanzen oder Substanzgruppen aufweist.

Die äußere Oberfläche der Teilchen des Trägermaterials weist eine Oberflächenmodifizierung $M_1$ auf, welche im wesentlichen keine Wechselwirkung mit Blutzellen zeigt, d.h. die äußere Oberfläche der Teilchen ist gegenüber Blutzellen sozusagen "glatt" bzw. inert.

Derartige, für Blutzellen glatte Oberflächenmodifizierungen $M_1$ sind in der Literatur bekannt. Die Oberflächenmodifizierung $M_1$ ist erfindungsgemäß vorzugsweise das Reaktionsprodukt der Umsetzung der Bindungsstellen auf den Oberflächen mit mindestens einer Poly(carbonsäure), Albumin, Heparin, Heparansulfat, Polyethylenoxid sowie Blockcopolymeren aus PE und Polypropylenoxid (PPO) und/oder einem Polymyxin. Besonders bevorzugt sind Polyacrylsäure und Heparin. Der Ausdruck Poly(meth)acrylsäure bzw. entsprechende Ausdrücke, wie sie in nachfolgend verwendet werden, stehen für solche Verbindungen, die von Acrylsäure oder von Methacrylsäure oder einem Gemisch davon abgeleitet sein können. Unter Poly(meth)acrylsäuren (PAS) werden durch radikalische Polymerisation von Acrylsäure und/oder Methacrylsäure anfallende Polymere der Formel $-(CH_2-C(H)(COOH))_n-$ und/oder $-(CH_2-C(CH_3)(COOH))_n-$ verstanden. Die Poly(meth)acrylsäuren sind jedoch auch durch Hydrolyse von polymeren (Meth)Acrylsäurederivaten, wie Estern, Amiden, Nitrilen, zugänglich. Eine erfindungsgemäß besonders geeignete Poly(meth)acrylsäure weist beispielsweise ein Gewichtsmittel des Molekulargewichts von $10^2$ g/Mol bis $10^7$ g/Mol auf. Polymyxine sind Peptidantibiotika, welche ausschließlich gegen Gram-negative Bakterien wirksam sind. Bei Polymyxin B handelt es sich um Cyclopeptide mit L-2,4-Diaminobuttersäure-Resten und einer D-Aminosäure.

Gemäß einer Ausführungsform kann die Oberflächenmodifizierung $M_1$ eine Wechselwirkung zu von Blutzellen verschiedenen Substanzen aufweisen. Dies ist möglich, solange die Oberflächenmodifizierung $M_1$ weiterhin keine Wechselwirkungen mit Blutzellen aufweist, sondem selektiv nur mit anderen, im Blut enthaltenen Substanzen wechselwirkt. Gemäß dieser Ausführungsform kann somit auch die Oberflächenmodifizierung $M_1$ eine funktionelle Oberflächenmodifizierung sein.

Die Oberfiächenmodifizierung $M_2$ wechselwirkt mit im Blut enthaltenen Substanzen, wobei nur solche Substanzen mit der Oberflächenmodifizierung $M_2$ in Kontakt treten können, welche abhängig von der gewählten Porengröße klein genug sind, um in die Poren einzudringen. Die Oberflächenmodifizierung $M_2$ ist somit eine sogenannte funktionelle Oberflächenmodifizierung, wobei unter funktionellen Oberflächenmodifizierungen beispielsweise Separationseffektoren oder auch Katalysatoren oder Enzyme verstanden werden. Separationseffektoren erzeugen selektive Wechselwirkungen, die für chromatographische Trennungen oder andere Verteilungsverfahren, wie der Flüssig-Flüssig-Verteilung, verwendet werden können.

Da die Blutzellen nicht in die Poren eindringen können und somit nicht in direkten Kontakt mit den Oberflächenmodifizierungen $M_2$ treten können, ist es möglich, die innere Oberfläche der Adsorbensteilchen mit einer Blutzellenunverträglichen Oberflächenmodifizierung $M_2$ zu versehen.

Die mindestens eine Oberflächenmodifizierung $M_2$ umfaßt vorzugsweise mindestens einen, auf der inneren Oberfläche vorliegenden Immunadsorber. Unter einem "Immunadsorber" wird erfindungsgemäß eine Verbindung verstanden, welche durch eine Immunreaktion befähigt ist, mit bestimmten Substanzen oder Substanzgruppen eine vorzugsweise spezifische Bindung einzugehen. Besonders bevorzugte Immunadsorber sind solche, welche aus der Gruppe, bestehend aus dem Komplementfaktor C1q, Amin-haltigen funktionellen Gruppen, Peptiden, Antikörpem, wie monoklonalen oder polyklonalen Anti-Fibrinogen- oder Anti-Fibrin-Antikörpem, und Antigenen, wie synthetischen A/B-Blutgruppenantigenen sowie DNA und RNA oder deren Spiegelmolekülen ausgewählt sind.

Wenn es sich bei der Oberflächenmodifizierung $M_1$ und/oder $M_2$ um funktionelle Gruppen handelt, können diese über einen Spacer kovalent mit dem Trägermaterial verbunden sein. Unter einem "Spacer" werden organische Brückenglieder mit verschiedener chemischer Struktur und Länge verstanden, mit Hilfe derer organische funktionelle Gruppen an die Oberfläche eines Trägermaterials gebunden werden können. Vorzugsweise werden jedoch derartige funktionelle Gruppen als Oberflächenmodifizierungen $M_1$ und/oder $M_2$ ohne einen Spacer direkt an eine Oberfläche des Trägermaterials gebunden. Durch die direkte Anbindung einer funktionellen Gruppe an das Trägermaterial kann ein Reaktionsschritt vennieden werden.

Somit können bei geeigneter Oberflächenmodifizierung $M_2$ an der inneren Oberfläche des Adsorbensmaterials in Vollblut enthaltene Substanzen abgetrennt werden, welche den etwa gleichen oder vorzugsweise einen kleineren Durchmesser als die gewählte mittlere Porengröße des porösen Trägermaterials aufweisen. Die mittlere Porengröße wird für eine effektive Trennung vorzugsweise so gewählt werden, daß die Poren wesentlich größer sind, als die abzutrennenden Teilchen.

Wichtig für den Einsatz eines derartigen Adsorbens ist die Möglichkeit der Sterilisierbarkeit, wie Gammabestrahlung, Plasmabehandlung oder Ethylenoxidbehandlung (insbesondere der Hitzesterilisierbarkeit z.B. bei 121 °C), da das behandelte Blut wieder dem Patienten zugeführt werden soll und keine Sepsis oder Entzündungen auslösen darf. Bevorzugt ist die Hitzesterilisierbarkeit bei 121°C und 1 bar Überdruck, gegebenenfalls in Verbindung mit einer stabilisierenden Vorbehandlung.

Durch das erfindungsgemäße Adsorbens ist die Abtrennung von in Blut enthaltenen Substanzen aus Vollblut möglich, auch wenn die zur Abtrennung erforderliche Oberflächenmodifizierung $M_2$ eine schädliche Wechselwirkung mit Blutzellen zeigen würde, da die Blutzellen nicht in die Poren des Adsorbens eindringen können, an deren Innenwänden die Oberflächenmodifizierung $M_2$ ausschließlich lokalisiert ist. Dadurch entfallen extrakorporale Schritte, wie die Abtrennung von Blutzellen, das Behandeln des isolierten Plasmas und das Zusammenführen der Blutbestandteile, wodurch die Biokompatibilität des Verfahrens gesteigert wird und beispielsweise die Gefahr einer Komplementaktivierung weiter erheblich verringert wird. Das Entfallen extrakorporaler Schritte bewirkt eine Verkürzung der Behandlungszeit und eine Vereinfachung des Verfahrens, wodurch eine Erhöhung der Sicherheit und des Wohlbefindens des Patienten erreicht wird.

Die vorliegende Erfindung betrifft ferner ein Verfahren zur Herstellung eines Adsorbens, welches die Schritte in der angegebenen Reihenfolge umfaßt:

- Bereitstellen eines teilchenförmigen, porösen organischen Trägermateriales mit einer mittleren Porengröße von $\leq$ 1,5 $\mu$m, wobei maximal 50% des Porenvolumens in Poren vorliegen kann, welche eine Porengröße von > 1,5 $\mu$m aufweisen, welches auf den äußeren und inneren Oberflächen modifizierbare Bindungsstellen aufweist,
- Füllen der Poren des Trägermaterials mit mindestens einem, unter den Bedingungen des Füllens flüssigen Medium, welches unter den Bedingungen des Modifizierens mit einer Lösung nicht mischbar ist, mit welcher Bindungsstellen auf den Oberflächen des Trägermaterials vollständig modifiziert werden können,
- vollständig Modifizieren der Bindungsstellen auf den äußeren Oberflächen des Trägermaterials zu mindestens einer Oberflächenmodifizierung $M_1$, die nicht mit Blutzellen wechselwirkt,
- gegebenenfalls Entfernen des Mediums, weiches unter den Bedingungen des Modifizierens mit einer Lösung, mit weicher Bindungsstellen auf den Oberflächen des Trägermaterials vollständig modifiziert werden können, nicht mischbar ist, aus den Poren, und
- Modifizieren der Bindungsstellen auf den inneren Oberflächen des Trägermaterials zu mindestens einer Oberflächenmodifizierung $M_2$ mit einer Reaktionslösung, welche zum Modifizieren der Oberflächenmodifizierungen $M_1$ nicht befähigt ist, wobei die Oberflächenmodifizierung $M_2$ mit in Blut enthaltenen Substanzen wechselwirkt.

Gemäß dem vorstehend beschriebenen Schritt des Füllens der Poren des Trägermaterials einer Ausführungsform des erfindungsgemäßen Verfahrens werden zunächst die Poren des Trägermaterials mit einem unter den Bedingungen des Füllens flüssigen Medium gefüllt. Dieses Medium ist unter den Bedingungen des Modifizierens nicht mit einer zum vollständigen Modifizieren von Bindungsstellen auf den Oberflächen des Trägermaterials zu mindestens einer Oberftächenmodifzierung $M_1$ befähigten Lösung mischbar.

Der Begriff unter den Bedingungen des Füllens flüssiges Medium" umfaßt erfindungsgemäß Medien, welche in die Poren eines Trägermaterials eingebracht bzw. eingefüllt werden können und aus diesen Poren wieder im wesentlichen vollständig entfernt werden können und welche unter den Bedingungen des Modifizierens nicht mit einer zum vollständigen Modifizierung von Bindungsstellen auf den Oberflächen des Trägermaterials zu mindestens einer Oberflächenmodifizierung $M_1$ befähigten Lösung mischbar sind. Derartige Medien können aus einem einzelnen Medium, aus Gemischen von mindestens zwei von diesen und/oder Lösungen bestehen.

Das Medium wird in der Regel eines sein, welches bei Raumtemperatur flüssig ist. Gemäß einer besonderen Ausführungsform kann jedoch das Medium auch bei Raumtemperatur gasförmig sein. Dann werden die Poren beispielsweise bei erniedrigter Temperatur und/oder erhöhtem Druck gefüllt und auch die Bindungsstellen auf den äußeren Oberflächen werden unter diesen Bedingungen modifiziert. Diese Ausführungsform bringt den Vorteil mit sich, daß ein bei Raumtemperatur gasförmiges Medium besonders leicht wieder aus den Poren entfernt werden kann. Gemäß einer weiteren Ausführungsform kann das Medium bei Raumtemperatur fest sein. Dann können die Poren des Trägermaterials beispielsweise unter Erwärmen gefüllt werden. Diese Ausführungsform weist den Vorteil auf, daß die Poren besonders dauerhaft, beispielsweise für längere Umsetzungen, mit dem Medium gefüllt sind.

In der Praxis kann dieses Füllen der Poren mit einem Medium durchgeführt werden, indem im einfachsten Fall die Teilchen des porösen Trägermaterials in ein bei Raumtemperatur flüssiges Medium eingetaucht und mit diesem aufgequollen werden, so daß die Luft im wesentlichen vollständig aus den Poren des Trägermaterials verdrängt wird. Ferner können die Teilchen auch in einen luftdichten Behälter gegeben werden, woraufhin die Luft aus dem Behälter und somit auch aus den Poren des porösen Trägermaterials durch Anlegen von Vakuum entfernt wird, und anschließend das Medium in den evakuierten Behälter unter Bedingungen, bei welchem dieses Medium flüssig ist, auf die Teilchen gegeben wird.

Nach dem Aufquellen der Teilchen kann das überschüssige flüssige Medium beispielsweise mit einer Nutsche von den Trägermaterialteilchen kurz abgesaugt werden, ohne daß das in den Poren enthaltene Medium wieder entfernt wird. Anschließend wird die Reaktionslösung zum Modifizieren der Bindungsstellen auf den Oberflächen des Trägermaterials zu einer Oberflächenmodifizierung $M_1$ zu den Teilchen gegeben.

Das Medium zum Füllen der Poren ist erfindungsgemäß unter den Bedingungen des Modifizierens nicht mit dieser Reaktionslösung mischbar. Somit wird während einer Modifizierung $M_1$ der äußeren Oberflächen bzw. bei Verwendung einer Reaktionslösung auf wässriger Basis vorzugsweise ein hydrophobes Medium als porenfüllendes bzw. porenbildendes Medium verwendet. Als hydrophobes Medium sind insbesondere solche Medien bevorzugt, welche aus der Gruppe, bestehend aus linearen oder verzweigten, acyclischen oder cyclischen $C_1$- bis $C_{20}$-Alkanen, wie Pentan, Hexan, Heptan, Octan, Nonan, Decan, Cyclohexan, und linearen oder verzweigten, acyclischen oder cyclischen $C_5$- bis $C_{20}$-Alkanoten, wie Hexanol, Octanol, Decanol, Undecanol und Dodecanol, linearen und verzweigten, acyclischen oder cyclischen $C_2$- bis $C_{30}$-Carbonsäureestem und aromatischen $C_6$- bis $C_{20}$-Kohlenwasserstoffen, sowie Mischungen davon ausgewählt sind.

Nach dem Füllen der Poren mit einem Medium, welches unter den Bedingungen des Modfizierens mit einer zur im wesentlichen vollständigen Modifizierung von Bindungsstellen auf den äußeren Oberflächen des Trägermaterials zu mindestens einer Oberflächenmodifizierung $M_1$ befähigten Lösung nicht mischbar ist, werden die Bindungsstellen auf den äußeren Oberflächen des Trägermaterials durch Umsetzen zu mindestens einer Obenlächenmodifizierung $M_1$ vollständig modifiziert.

Erfindungsgemäß bedeutet der Begriff "vollständige Modifizierung", daß im wesentlichen alle auf einer Oberfläche des Trägermaterials vorhandenen Bindungsstellen entweder der äußeren Oberfläche oder der inneren Oberfläche zu Oberfiächenmodiflzierungen $M_1$ bzw. $M_2$ umgesetzt werden und somit im wesentlichen keine freien bzw. modifizierbaren Bindungsstellen mehr auf der jeweiligen Oberfläche des Trägermaterials verbleiben. Um eine derartige, vollständige Modifizierung zu bewirken, enthalten die jeweiligen Reaktionslösungen vorzugsweise jeweils einen Überschuß Modifizierungsmittel.

Der Begriff "Modifizieren'' umfaßt erfindungsgemäß das Verändern, Umsetzen, Schaffen und/oder Zerstören von Bindungsstellen auf der jeweiligen Oberfläche des porösen Trägermaterials. Gemäß einer bevorzugten Ausführungsform bedeutet der Begriff "Modifizieren" das Umsetzen von Bindungsstellen auf den Oberflächen des porösen Trägermaterials mit beispielsweise Nukleophilen oder Elektrophilen zu Oberflächenmodifizierungen $M_1$ und/oder $M_2$. Oberflächenmodifizierungen $M_1$ weisen erfindungsgemäß vorzugsweise die Eigenschaft auf, daß sie nicht mit Blutzellen wechselwirken, wohingegen Oberflächenmodifizierungen $M_2$ mit in der aufzutrennenden Lösung, Suspension oder Dispersion, wie beispielsweise Blut, vorliegenden Substanzen wechselwirken.

Nach dem vollständigen Modifizieren der Bindungsstellen auf den äußeren Oberflächen des Trägermaterials zu mindestens einer Oberflächenmodifizierung $M_1$ wird die für die Modifizierung benötigte Reaktionslösung und anschließend gegebenenfalls auch das Medium, welches mit einer zum Modifizieren der Bindungsstellen auf den äußeren Oberflächen des Trägermaterials zu mindestens einer Oberflächenmodifizierung $M_1$ befähigten Lösung nicht mischbar ist, aus den Poren entfernt, beispielsweise durch Absaugen, Erwärmen, gegebenenfalls längeres Anlegen von Vakuum und/oder Herauswaschen mit einem leicht entfembaren, weiteren Medium.

Gemäß der vorliegenden Erfindung werden nach dem Modifizieren der Bindungsstellen auf den äußeren Oberflächen des porösen Trägermaterials die Bindungsstellen auf den inneren Oberflächen des porösen Trägermaterials modifiziert. Falls die Reaktionslösung für die Oberflächenmodifizierung $M_2$ mit dem in den Poren vorliegenden Medium mischbar ist, kann das Medium in den Poren verbleiben und muß nicht notwendigerweise entfernt werden, da durch Mischen des Mediums in den Poren mit der Reaktionslösung zur Modifizierung der Bindungsstellen zu Oberflächenmodifizierungen $M_2$ und Diffusion dieser Reaktionslösung in die Poren auch die Bindungsstellen in den Poren modifiziert werden können. Meistens wird es aber bevorzugt sein, das Medium zunächst aus den Poren zu entfernen. Auch die inneren Oberflächen des Trägermaterials werden vorzugsweise vollständig zu mindestens einer Oberflächenmodifizierung $M_2$ modifiziert.

Gemäß einer anderen Ausführungsform können die Oberflächenmodifizierungen $M_2$ auch schon vor dem Modifizieren der Bindungsstellen auf den äußeren Oberflächen zu Oberflächenmodifizierungen $M_1$ auf den Oberflächen des porösen Trägermaterials vorliegen. Gemäß dieser Ausführungsform können, wenn erforderlich, zunächst alle Bindungsstellen auf den inneren und äußeren Oberflächen zu Oberflächenmodifizierungen $M_2$ umgesetzt werden. Es kann aber auch sein, daß die Bindungsstellen des Trägermaterials auch schon ohne Modifizierung die erforderlichen Bindungseigenschaften von $M_2$ aufweisen. Ein derartiges, Gruppierungen $M_2$ tragendes, gegebenenfalls modifiziertes Trägermatedal wird als Trägermaterial in dem erfindungsgemäßen Verfahren in dem Schritt des Bereitstellens eines teilchenförmigen, porösen Trägermaterials, welches auf den äußeren und inneren Oberflächen modifizierbare Bindungsstellen aufweist, bereitgestellt. Anschließend werden die Schritte des Füllens der Poren des Trägermaterials mit mindestens einem, unter den Bedingungen des Füllens flüssigen Medium, welches unter den Bedingungen des Modifizierens mit einer Lösung nicht mischbar ist, mit welcher Bindungsstellen auf den Oberflächen des Trägermaterials vollständig modifiziert werden können; des vollständigen Modifizierens der Bindungsstellen auf den äußeren Oberflächen des Trägermaterials zu mindestens einer Oberflächenmodifizierung $M_1$ und des gegebenenfalls Entfemens des Mediums, welches unter den Bedingungen des Modifizierens mit einer Lösung, mit welcher Bindungsstellen auf den äußeren Oberflächen des Trägermaterials vollständig modifiziert werden können, nicht mischbar ist, aus den Poren gemäß einer Ausführungsform des erfindungsgemäßen Verfahrens durchgeführt, wobei in dem Schritt des vollständigen Modifizierens der Bindungsstellen auf den äu-ßeren Oberflächen des Trägermaterials zu mindestens einer Oberflächenmodifizierung $M_1$ die Modifizierung der modifizierbaren Bindungsstellen auf den äußeren Oberflächen des Trägermaterials beispielsweise auch durch Zerstören der Ober flächenmodifizierungen $M_2$ durchgeführt werden kann.

Gemäß einer weiteren Ausführungsform ist es auch möglich das erfindungsgemäße Adsorbens herzustellen, indem nach dem Bereitstellen eines teilchenförmigen porösen Trägermaterials, weiches auf den äußeren Oberflächen und den inneren Oberflächen modifizierbare Bindungsstellen aufweist, die Bindungsstellen auf den äußeren Oberflächen des Trägermaterials vollständig zu Oberflächenmodifizierungen $M_1$ umgesetzt werden, wobei diese Umsetzung durch eine das Trägermaterial nicht durchdringende Strahlung bewirkt wird. Da eine derartige Strahlung nicht in die Poren eindringen kann, findet in den Poren keine Umsetzung der Bindungsstellen auf dem Trägermaterial zu Oberflächenmodifizierungen $M_1$ statt. Im Anschluß daran können gegebenenfalls Bindungsstellen auf den inneren Oberflächen des Trägermaterials zu Oberflächenmodifizierungen $M_2$ umgesetzt werden, wobei diese Umsetzung ohne die vorstehende Strahlung bewirkt wird.

Somit umfaßt ein Verfahren gemäß dieser Ausführungsform die folgenden Schritte in der angegebenen Reinhenfolge :

- Bereitstellen eines teilchenförmigen, porösen organischen Trägermaterials mit einer mittleren Porengröße von $\leq$ 1,5 $\mu$m, wobei maximal 50% des Porenvolumens in Poren vorliegen kann, welche eine Porengröße von > 1,5 $\mu$m aufweisen, welches auf den äußeren Oberflächen und den inneren Oberflächen modifizierbare Bindungsstellen aufweist, welche zu Oberflächenmodifizierungen $M_1$ und/oder $M_2$ umgesetzt werden können,
- vollständiges Modifizieren der Bindungsstellen auf den äußeren Oberflächen des Trägermaterials zu mindestens einer Oberflächenmodifizierung $M_1$, die nicht mit Blutzellen wechselwirkt, mittels einer Umsetzung, welche durch eine das Trägermaterial nicht durchdringende Strahlung bewirkt wird, und
- vollständiges Modifizieren der Bindungsstellen auf den inneren Oberflächen des Trägermaterials zu mindestens einer Oberflächenmodifizierung $M_2$ mittels einer Umsetzung, welche zum Modifizieren der Oberflächenmodifizierung $M_1$ nicht befähigt ist, wobei die Oberflächenmodifizierung $M_2$ mit in Blut enthaltenen Substanzen wechselwirkt.

Vorzugsweise ist die das Trägermaterial nicht durchdringende Strahlung elektromagnetische Strahlung im UV- oder sichtbaren Bereich.

Auch gemäß diesem Verfahren ist es möglich, daß das bereitgestellte poröse Trägermaterial bereits auf allen Oberflächen eine modifizierbare Oberflächenmodifizierung $M_2$ aufweist und daß nach dem vollständigen Modifizieren der Bindungsstellen auf den äußeren Oberflächen das Modifizieren der Bindungsstellen auf den inneren Oberflächen nicht mehr erforderlich ist.

Nach einer bevorzugten Ausführungsform der vorliegenden Erfindung kann das vorstehend beschriebene poröse Trägermaterial, welches zu einem erfindungsgemäßen Adsorbens modifiziert werden kann, wie eingangs erwähnt, ausgehend von bestimmten polymerisierbaren Verbindungen, durch das nachfolgend beschriebene Suspenslonspolymerisationsvertahren hergestellt werden.

Dieses Verfahren zur Herstellung eines Adsorbens umfaßt die folgenden Schritte in der angegebenen Reihenfolge :

- Herstellen eines teilchenförmigen, porösen Trägermatenals mit einer mittleren Porengröße von $\leq$ 1,5 $\mu$m, wobei maximal 50% des Porenvolumens in Poren vorliegen kann, welche eine Porengröße von < 1,5 $\mu$m aufweisen, welches auf den äußeren und inneren Oberflächen modifizierbare Bindungsstellen aufweist, durch Suspensionspolymerisation, wobei eine hydrophobe Phase, umfassend mindestens ein Monomer, mindestens einen Vemetzer, ein porenbildendes Medium und einen Polymerisationsinitiator, in einer kontinuierlichen Phase, umfassend Wasser und mindestens ein Schutzkolloid, dispergiert wird, und die sich in der hydrophoben Phase befindlichen Monomere und Vemetzer polymerisiert werden, wobei die Poren des resultierenden teilchenförmigen porösen Trägermaterials mit dem porenbildenden Medium gefüllt sind,
- vollständiges Modifizieren der modifizierbaren Bindungsstellen auf den äußeren Oberflächen der Trägermaterialteilchen zu mindestens einer Oberflächenmodifizierung $M_1$, die nicht mit Blutzellen wechselwirkt,

- gegebenenfalls Entfernen des porenbildenden Mediums aus den Poren, und
- Modifizieren der Bindungsstellen auf den inneren Oberflächen des Trägermaterials zu mindestens einer Oberflächenmodifizierung $M_2$ mit einer Lösung, welche zum Modifizieren der Oberflächenmodifizierung $M_1$ nicht befähigt ist, wobei die Oberflächenmodifizierung $M_2$ mit in Blut enthaltenen Substanzen wechselwirkt.

Nach einer weiteren bevorzugten Ausführungsform der vorliegenden Erfindung kann das poröse Trägermaterial, welches zu einem erfindungsgemäßen Adsorbens modifiziert werden kann, auch durch das nachfolgend beschriebene Suspensionspölymensationsverfahren hergestellt werden, wobei zu der wässrigen kontinuierlichen Phase ein wasserlösliches Monomer zugegeben wird, wodurch die Herstellung der porösen Teilchen des Trägermaterials und die Modifizierung der äußeren Oberflächen dieser Teilchen in einem Schritt erfolgen.

Dieses weiterhin bevorzugte Verfahren zur Herstellung eines Adsorbens umfaßt die folgenden Schritte in der angegebenen Reihenfolge :

- Herstellen eines teilchenförmigen, porösen Trägermaterials mit einer mittleren Porengröße von $\leq 1,5\ \mu m$, wobei maximal 50% des Porenvolumens in Poren vorliegen kann, welche eine Porengröße von $< 1,5\ \mu m$ aufweisen, welches auf den äußeren und inneren Oberflächen modifizierbare Bindungsstellen aufweist, durch Suspensionspolymerisation, wobei eine hydrophobe Phase, umfassend mindestens ein Monomer, mindestens einen Vemetzer, ein porenbildendes Medium und einen Polymerisationsinitiator,
in einer kontinuierlichen Phase, umfassend Wasser, mindestens ein Schutzkolloid und mindestens ein wasserlösliches, in der hydrophoben Phase unlösliches Monomer, dispergiert wird, wobei die sich in der hydrophoben Phase befindlichen Monomere und Vemetzer unter Ausbildung eines porösen Kerns polymerisiert werden und das mindestens eine, wasserlösliche Monomer in der Phasengrenzfläche zwischen hydrophober und kontinuierlicher Phase zu mindestens einer Oberflächenmodifizierung $M_1$ polymerisiert, die nicht mit Blutzellen wechselwirkt, wobei mindestens ein Teil der sich aus dem wasserlöslichen Monomer bildenden Polymerketten kovalent an den sich bildenden porösen Kem gebunden wird,
- gegebenenfalls Entfernen des porenbildenden Mediums aus den Poren, und
- Modifizieren der Bindungsstellen auf den inneren Oberflächen des Trägermaterials zu mindestens einer Oberflächenmodifizierung $M_2$ mit einer Lösung, welche zum Modifizieren der Oberflächenmodifizierung $M_1$ nicht befähigt ist, wobei die Oberflächenmodifizierung $M_2$ mit in Blut enthaltenen Substanzen wechselwirkt.

In den vorstehend beschriebenen Suspensionspolymerisationsverfahren können in der wässrigen kontinuierlichen Phase auf dem Fachgebiet übliche Schutzkolloide bzw. Stabilisatoren verwendet werden. Vorzugsweise werden ein oder mehrere Schutzkolloide aus der Gruppe Polyvinylalkohol (PVA), Polyvinylpyrrolidon (PVP) und Polyethylengylkol verwendet.

Das in der hydrophoben Phase enthaltene Monomer ist vorzugsweise ein Epoxidgruppen enthaltendes Monomer oder ein Gemisch aus einem Epoxidgruppen enthaltenden Monomer und mindestens einem weiteren Monomer. Wie vorstehend beschrieben können jedoch auch andere Monomere, die ein gespanntes heterocyclisches System als funktionelle Gruppe tragen, beispielsweise Aziridingruppen oder Episulfdgruppen, verwendet werden. Das Epoxidgruppen enthaltende Monomer ist vorzugsweise ein Epoxidgruppen enthaltendes (Meth)Acrylsäuremonomer. Unter einem Epoxidgruppen enthaltenden (Meth)Acrylsäuremonomer versteht man in der vorliegenden Erfindung vor allem eines, bei dem der Rest, welcher die Epoxidgruppe trägt, über die Esterfunktjonalität einer substituierten oder unsubstituierten (Meth)Acrylsäure gebunden ist. Es ist jedoch auch denkbar, daß der Rest, welcher die Epoxidgruppe trägt, in einer anderen Art und Weise an das (Meth)Acrylsäuremonomer gebunden ist, beispielsweise an eines der Kohlenstoffatome der C-C-Doppelbindung. Nach einer besonders bevorzugten Ausführungsform ist das Epoxidgruppen enthaltende (Meth) Acrylsäuremonomer Gfycidyl(meth)acrylat.

In den vorstehend beschriebenen Suspensionspolymerisationsverfahren wird als Vemetzer ein Monomer mit mindestens zwei polymerisierbaren Gruppen verwendet; welches zu einer Quervemetzung der wachsenden Polymerketten führt. Unter dem Ausdruck "Vemetzer" versteht man übliche auf dem Fachgebiet verwendete Monomere mit mindestens zwei polymerisierbaren Gruppen, wobei die polymerisierbaren Gruppen beispielsweise Vinyl-, Acrylat- oder Methacrylatgruppen sein können. Vorzugsweise wird als Monomer mit mindestens zwei polymerisierbaren Gruppen ein Monomer auf Basis von (Meth)Acrylsäure verwendet. Nach einer besonders bevorzugten Ausführungsform ist das Monomer mit mindestens zwei polymerisierbaren Gruppen Ethylenglykoldi(ineth)acrylat: Ferner können die beiden (Meth)acrylatgruppen eines derartigen Monomers auch durch eine andere, von beispielsweise $\alpha,\omega$-Diolen oder allgemein Alkoholen mit mindestens zwei OH-Gruppen abgeleitete Verknüpfungsgruppe, verbunden sein. Der Anteil des Vernetzers, bezogen auf den Gehalt an Monomer in der hydrophoben Phase, kann von 5 bis 95 Gew.-%, vorzugsweise 20 bis 60 Gew.-%, besonders bevorzugt 40 Gew.-%, betragen.

In den vorstehend beschriebenen Suspensionspolymerisationsverfahren ist das porenbildende Medium ein Gemisch aus mindestens zwei Komponenten, ausgewählt aus linearen, verzweigten oder cyclischen Alkoholen mit 5 bis 14 Kohlenstoffatomen und Estern von Mono- oder Dicarbonsäuren mit 2 bis 10 Kohlenstoffatomen, die mit ein- oder mehrwertigen Alkoholen mit 1 bis 6 Kohlenstoffatomen verestert sind. Eine Komponente des vorstehend beschriebenen porenbildenden Mediums kann ein linearer, verzweigter oder cyclischer (primärer, sekundärer oder tertiärer) Alkohol mit 5 bis 14 Kohlenstoffatomen, beispielsweise Hexanol, Octanol, Decanol oder Dodecanol, sein. Eine weitere Komponente des vor stehend beschriebenen porenbildenden Mediums kann ein Ester einer Mono- oder Dicarbonsäure mit 2 bis 10 Kohlenstoffatomen sein, die mit ein- oder mehrwertigen Alkoholen mit 1 bis 6 Kohlenstoffatomen verestert ist, beispielsweise Essigsäurebutylester, Bemsteinsäurediethylester oder Glycerintributtersäureester. Als porenbildendes Medium bzw. als Porenbildner wird ein Gemisch aus zwei Komponenten verwendet, wobei das resultierende Polymer in keiner der Komponenten löslich ist, jedoch eine Komponente das während der Suspensionspolymerisation sich bildende Oligomer löst und die andere Komponente nicht befähigt ist, das sich bildende Oligomer zu lösen. Aufgrund der spezifischen Auswahl der Komponenten des porenbildenden Mediums wird eine Porenbildung ermöglicht. Die vorstehend beschriebene Methode zur Erzeugung von Poren in einem polymeren Trägermaterial ist einem Fachmann bekannt. Ferner kann in Routineversuchen, beispielsweise durch geeignete Auswahl des porenbildenden Mediums bzw. durch Variieren des Verhältnisses der Komponenten, die Porengröße, das Porenvolumen und der Porendurchmesser in gewünschter Art und Weise eingestellt werden. In diesem Zusammenhang versteht man unter dem Ausdruck "Oligomer" ein sich in einer vorstehend beschriebenen Suspensionspolymerisation bildendes Material mit einem Molekulargewicht bis etwa $10^4$ g/mol. Besonders bevorzugt ist als porenbildendes Medium ein Gemisch aus Dodecanol und Cyclohexanol bzw. ein Gemisch aus Bemsteinsäurediethylester und Dodecanol. Der Anteil des vorstehend beschriebenen porenbildenden Mediums, bezogen auf die hydrophobe Phase, kann von 20 bis 90 Gew.-%, vorzugsweise von 40 bis 80 Gew.-%, besonders bevorzugt 70 Gew.-%, betragen.

Ein in den vorstehend beschriebenen Suspensionspolymerisationsverfahren zu verwendender Polymerisationsinitiator unterliegt im allgemeinen keinen besonderen Beschränkungen. Beispielsweise kann ein Polymerisationsinitiator aus der Gruppe von Azoverbindungen, Peroxiden und Redoxinitiatoren verwendet werden. Vorzugsweise wird als Polymerisationsinitiator eine dafür geeignete Azoverbindung oder ein dafür geeignetes Peroxid eingesetzt. Besonders bevorzugt ist der Polymerisationsinitiator Azobisisobutyronitril oder Dibenzoylperoxid. Der Anteil des Polymerisationsinitiators, bezogen auf die Monomermenge, kann von 0,1 bis 5 Gew.-%, vorzugsweise 0,5 bis 2 Gew.-%, besonders bevorzugt 1 Gew.-%, betragen.

In dem vorstehend beschriebenen Suspensionspolymerisationsverfahren, bei dem in der wässrigen kontinuierlichen Phase ein wasserlösliches Monomer zugegeben wird, kann das wasserlösliche Monomer eine monofunktionale Vinylverbindung sein. Vorzugsweise weist das wasserlösliche Monomer ein oder mehrere polare Gruppen auf. Besonders bevorzugt ist das wasserlösliche Monomer ausgewählt aus: Hydroxyalkylmethacrylaten, Aminoalkylmethacrylaten, N-Vinyl-2-pyrrolidon, Acryl- und Methacrylnitril, Acryl- und Methacrylsäure, Allylalkohol, Allylamin, 3-Allyloxy-1,2-propandiol, (Ethylenglykol)n-vinylmethylether mit n=1 bis 10, N-Methylolacrylamid, 2-Acrylamido-2-methylpropansulfonsäure und Salzen der Vinylbenzolsulfonsäure, Vinylsulfonsäure, sowie Kombinationen davon. Als Hydroxyalkylmethacrylate und/oder Aminoalkylmethacrylate sind solche bevorzugt, die eine Alkylgruppe von 1 bis 10 Kohlenstoffatomen aufweisen, beispielsweise eine Methyl-, Ethyl-, Propyl- oder Butylgruppe. Noch mehr bevorzugt ist das wasserlösliche Monomer Acrylsäure, N-Vinyl-2-pyrrolidon oder eine Kombination davon. Der Anteil des wasserlöslichen Monomers kann 2 Gew.-% bis 80 Gew.-%, bezogen auf das Gesamtausgangsmonomergewicht des porösen Trägermaterials, betragen. Der Anteil des wasserlöslichen Monomers beträgt vorzugsweise 5 Gew.-% bis 50 Gew.-%, bezogen auf das Gesamtausgangsmonomergewicht des porösen Trägermaterials, besonders bevorzugt 20 bis 30 Gew.-%.

Die vorliegende Erfindung betrifft ferner die Verwendung des erfindungsgemäßen Adsorbens zum Abtrennen von Biomolekülen und/oder pathogenen Stoffen, wie Toxinen, aus Vollblut. Beispielsweise können Biomakromoleküle, wie z.B. LDL und Endotoxine, Immunglobuline, Fibrin, Fibrinogene, Immunkomplexe, Exotoxine, Fibronectine und/oder Superantigene entfernt werden.

Ferner stellt die vorliegende Erfindung die Verwendung des erfindungsgemäßen Adsorbens zur Herstellung eines Adsorbers, umfassend ein Gehäuse und darin befindliches Adsorbens, zum Abtrennen von Biomolekülen und/oder pathogenen Stoffen aus Vollblut bereit. Ein mit dem erfindungsgemäß hergestellten Adsorbens ausgerüsteter Adsorber weist ein Gehäuse auf, welches vorzugsweise in Röhren- oder Säulenform ausgebildet ist, und welches das Adsorbens als Füllmaterial enthält. Im Hinblick auf die üblicherweise durchzusetzenden Blutmengen und die Effizienz des erfindungsgemäßen Adsorbers umfaßt der Adsorber vorzugsweise ein Volumen von 250 bis 1250 ml. Der Adsorber kann einzeln oder im Doppel- oder Mehrfachbetrieb eingesetzt werden. Bei zwei oder mehr Adsorbern besteht die Möglichkeit, abwechselnd einen Adsorber mit dem Blut zu beschicken, während der andere Adsorber regeneriert wird. Dies führt zu einer weiteren Effizienz beim Einsatz des erfindungsgemäß hergestellten Adsorbens. Der das Adsorbens enthaltende Adsorber ist vorzugsweise so ausgebildet, daß er ein Gehäuse mit einem kopfseitigen Einlaßbereich aufweist, durch den das Blut dem Adsorber zugeführt wird, wobei sich in diesem Fall der Auslaß am Boden des Gehäuses des Adsorbers befindet.

Um zu verhindern, daß unerwünschte Substanzen, z.B. Substanzen, die vom Adsorbensmaterial herrühren, mit dem behandelten Blut in den Blutkreislauf des Patienten zurückgeführt werden, befindet sich am Auslaß des Gehäuses des Adsorbers vorzugsweise ein Filter. Dabei handelt es sich vorzugsweise um einen Partikelfilter.

Die vorliegende Erfindung wird durch die nachfolgenden Beispiele weiter erläutert.

**Beispiele**

Beispiel 1

Reaktionslösung. I: 75 mg CMECDI (2-Morpholino-ethansulfonsäure Monohydrat) wurden bei 4°C in 200 ml 0,1 M MES (N-Cyclohexyl-N'-(2-morpholinoethyl)-carbodiimid-methyl-p-toluolsulfat) mit einem pH von 4,75 gelöst. Es wurden 75 mg Polyacrylsäure mit einem gewichtsgemittelten Molekulargewicht von 800.000 zugegeben, der pH auf 4,75 eingestellt und bei 4°C 0,5 Stunden gerührt.

In einem Rundkolben wurden 75 ml Trägermaterialteilchen (Eupergit C 250L) in 75 ml 25%iger $NH_3$-Lösung über Nacht gerührt. Die Trägermaterialteilchen wurden dreimal mit Wasser gewaschen, dekantiert, abgesaugt, nochmals mit Wasser gewaschen und getrocknet.

Die Trägermaterialteilchen wurden 30 Minuten in 100 ml n-Hexan quellen gefasst, 15 Sekunden auf einer Nutsche abgesaugt und danach sofort bei 4°C in die oben beschriebene Reaktionslösung I gegeben, geschüttelt und über Nacht bei 4°C gerührt. Anschließend wurden die Teilchen abgesaugt, mit Wasser, 4 M NaCl und wieder mit Wasser gewaschen und im Vakuumexsikkator über $CaCl_2$ getrocknet.

40 ml von diesen Teilchen wurden mit 50 ml 0,25%iger Glutardialdehydlösung zwei Stunden bei 40°C gerührt. Dann wurden die Teilchen mit 7,5 1 Wasser gewaschen und trockengesaugt.

Zu 18 ml einer C1q-Lösung wurden 32 ml einer mit Phosphat gepufferten Kochsalzlösung ("phosphate buffered saline", PBS) (0,1 M $NaH_2PO_4$, 0,154 M NaCl, pH 6,8) gegeben und die UV-Absorption bei 280 nm gemessen. Die bei 280 Nanometer gemessene Absorption betrug 1,040, entsprechend einer Konzentration von 1,53 g/Liter (Reaktionslösung II).

Zu dieser Lösung wurden die mit der Reaktionslösung I behandelten Teilchen gegeben; 3 Stunden bei Raumtemperatur gerührt und abgesaugt. Die UV-Absorption bei 280 Nanometer der Lösung betrug 0,577, entsprechend einer Konzentration von 0,85 g/Liter. Somit betrug die Kopplungsausbeute der Reaktionslösung II 0,68 g/Liter.

Danach wurden die Teilchen mit 600 ml PBS gewaschen, zweimal in 200 ml 5 mM Ascorbinsäure in PBS je eine Stunde bei Raumtemperatur gerührt und abgesaugt. Die Teilchen wurden 4 Stunden in 1 M Ethanolamin in PBS bei einem pH-Wert von 8 bei Raumtemperatur gerührt und abgesaugt. Anschließend wurden die Teilchen noch zweimal in 200 ml 5 mM Ascorbinsäure in PBS je eine Stunde bei Raumtemperatur gerührt und abgesaugt. Dann wurden die Teilchen mit folgenden Lösungen gewaschen:

1. 400 ml 0,1 M Acetat, 0,5 M NaCl, pH 4
2. 400 ml, 1 M $NaH_2PO_4$, 0,5 M NaCl, pH 9
3. 400 ml, 0,1 M $NaH_2PO_4$, 0,154 M NaCl, pH 6,8
4. 210,154 M NaCl.

Zuletzt wurden die Teilchen zur Hitzesterilisation 20 Minuten bei 121°C in 0,154 M NaCl autoklaviert.

Beispiele 2 bis 6

Adsorbensmaterialien wurden auf die gleiche Weise wie in Beispiel 1 hergestellt, wobei das verwendete Medium, Oberflächenmodiflzierungen, usw. in Tabelle 1 aufgeführt sind.

Zum Vergleich wurden Adsorbentien hergestellt, welche sowohl auf den inneren als auch den äußeren Oberflächen C1q (Vergleichsbeispiel 1), PAS (Vergleichsbeispiel 2) oder OH-Gruppen (Vergleichsbeispiel 3) als Oberflächenmodifizierungen trugen.

**Tabelle 1**

|  | $M_1$ | $M_2$ | Trägermaterial | Verfahren (Medium) | CMECDI-Aktivierung |
|---|---|---|---|---|---|
| Bsp. 1 | PAS | C1q | Beads 1 | I (Hexan) | nein |
| Bsp. 2 | PAS | C1q | Beads 1 | I (Dodecanol) | nein |
| Bsp. 3 | PAS | C1q | Beads 1 | I (Hexan) | ja |
| Bsp. 4 | PAS | C1q | Beads 1 | I (Dodecanol) | ja |
| Bsp. 5 | PAS | C1q | Beads 2 | I (Hexan) | ja |

Tabelle fortgesetzt

|  | $M_1$ | $M_2$ | Trägermaterial | Verfahren (Medium) | CMECDI-Aktivierung |
|---|---|---|---|---|---|
| Bsp. 6 | PAS | C1q | Beads 2 | I (Dodecanol) | ja |
| Vgl.-Bsp. 1 | C1q | C1q | Beads 1 | - | - |
| Vgl.-Bsp. 2 | PAS | PAS | Beads 1 | - | - |
| Vgl.-Bsp. 3 | OH | C1q | Beads 1 | - | - |

Anmerkungen:

- Alle Beads wiesen mittlere Porengrößen zwischen 100 und 200 nm auf.
- Als Beads 1 wurde Eupergit 250 L verwendet.
- Als Beads 2 diente ein durch Polymerisation von Ethylenglykoldi(meth)acrylat und Glycidylmethacrylat und Allylglycidylether hergestelltes, statistisches Copolymer.

Die Ergebnisse der Untersuchungen hinsichtlich der Bindungsfähigkeit für Thrombozythen bzw. Immunkomplexe sind in Tabelle 2 aufgeführt.

**Tabelle 2**

|  | $M_1$ | $M_2$ | Bindungsfähigkeit für | |
|---|---|---|---|---|
|  |  |  | Immunkomplexe | Thrombozythen |
| Bsp. 1 | PAS | C1q | ja | nein |
| Bsp. 2 | PAS | C1q | ja | nein |
| Bsp.3 | PAS | C1q | ja | nein |
| Bsp. 4 | PAS | C1q | ja | nein |
| Bsp. 5 | PAS | C1q | ja | nein |
| Bsp.6 | PAS | C1q | ja | nein |
| Vgl.-Bsp. 1 | C1q | C1q | ja | ja |
| Vgl.-Bsp. 2 | PAS | PAS | nein | nein |
| Vgl.-Bsp. 3 | OH | C1q | ja | ja |

Aus dieser Tabelle 2 ist ersichtlich, daß alle erfindungsgemäßen Adsorber gemäß den Beispielen 1 bis 6 eine Bindungsfähigkeit für Immunkomplexe aufweisen, aber nicht mit Thrombozythen wechselwirken. Ein Adsorbens, welches auch an den äußeren Oberflächen C1q-Oberflächenmodifizierungen aufwies, filterte neben Immunkomplexen auch Thrombozythen aus dem Vollblut heraus (Vergleichsbeispiel 1), so daß dieser Adsorber nicht für die Reinigung von Vollblut geeignet ist. Ein Adsorbens, welches nur PAS als Oberflächenmodifizierung aufwies (Vergleichsbeispiel 2), war zwar vollblutverträglich; wies jedoch keine Bindungsstellen für Immunkomplexe auf, so daß eine Abtrennung von Immunkomplexen nicht möglich war. Auch ein Adsorbens, welches OH-Gruppen als Oberflächenmodifizierung $M_1$ trug, zeigte eine, wenn auch schwache, Wechselwirkung mit Thrombozythen. Aus diesem Grund ist eine von einer OH-Gruppe verschiedene Oberflächenmodifizierung $M_1$ gemäß der vorliegenden Erfindung bevorzugt.

Beispiel 7

Schritt A (Herstellung eines porösen Trägermaterials durch Suspensionspolymerisation)

Ein poröses Trägermaterial wird beispielsweise gemäß EP 0 495 107 A1 durch Suspensionspolymerisation hergestellt. Das erhaltene Trägermaterial (Beads) wird mit einem Überdruck von 0,5 bar innerhalb von 30 s von der Mutterlauge abfiltriert.

Schritt B (Aminierung der äußeren Oberfläche des Trägermaterials)

100 g des feuchten Trägermaterials aus Schritt A wurden mit 300 ml 25 Gew.-%igem Ammoniak (FLUKA) für 3h bei Raumtemperatur auf einem Rotationsschüttler inkubiert. Anschließend wurde die Reaktionslösung mit 0,5 bar Überdruck vom Trägermaterial abfiltriert und dieses bis zur Neutralität mit destilliertem Wasser gewaschen.

Schritt C (Modifizierung der äußeren Oberfläche des Trägermaterials mit einer Oberflächenmodifizierung $M_1$)

25 g einer 10 Gew.-%igen Polyacrylsäurelösung (Degapas 11105S, Fa. Stockhausen) wurden in 175 g einer isotonischen NaCl-Lösung gelöst, auf pH = 4,75 eingestellt und mit einer Lösung aus 1,25 g N-(3-Dimethylaminopropyl)-N'-ethylcarbodiimidhydrochlorid (FLUKA) in 50 ml einer isotonischen NaCl-Lösung für 45 min bei Raumtemperatur auf dem Rotationsschüttler inkubiert.
Anschließend wurde das abfiltrierte Trägermaterial aus Schritt B zu der Lösung gegeben und für 4h bei Raumtemperatur auf dem Rotationsschüttler inkubiert.

Schritt D (Freiwaschen des Porenraums des Trägermaterials)

Nach dem Absaugen der vorstehend beschriebenen Modifizierungslösung wird das Trägermaterial einmal mit 600 ml einer 0,05 M Natriumhydroxidlösung in 50%igem Isopropanol, 4 mal mit 600 ml Isopropanol und 2 mal mit 600 ml destilliertem Wasser gewaschen.

Schritt E (Aminierung der inneren Oberfläche des Trägermaterials)

Die Aminierung der inneren Oberfläche des nach Schritt D erhaltenen Trägermaterials erfolgt analog zu Schritt B jedoch mit 12,5%igem Ammoniak bei 40°C (Reaktionszeit 4h).

Schritt F (Kuppeln der Aminogruppen mit der Oberflächenmodifizierung $M_2$)

Das Trägermaterial aus Schritt E wird 4 mal mit 250 ml $NaH_2PO_4$-Puffer (pH = 6,8) (PP-Puffer) gewaschen. Anschließend wird das Trägermaterial für 2h bei 40°C mit 300ml einer 0,4%igen Glutardialdehydlösung (FLUKA) in PP-Puffer auf dem Rotationsschüttler inkubiert, 12 mal mit jeweils 1l destilliertem Wasser gewaschen und 4 mal mit 1l PP-Puffer auf pH = 6,8 äquilibriert. Dann wurden die Glutardialdehydgruppen mit 300 ml einer Ethanolaminlösung (FLUKA) in destilliertem Wasser bei einem pH = 8 (4h, Raumtemperatur, Rotationsschüttler) umgesetzt und das Trägermaterial 4 mal mit je 250 ml PP-Puffer gewaschen. Anschließend wurde das Trägermaterial mit 250 ml einer 5 mM Ascorbinsäurelösung (FLUKA) in PP-Puffer für 4h bei Raumtemperatur auf dem Rotationsschüttler inkubiert (im Dunkeln) und mit jeweils 500 ml:

- 0,1 M Natriumacetatpuffer, pH = 4,6, in isotonischer NaCl-Lösung
- 0,1 M Natriumphosphatpuffer, pH = 9, in isotonischer NaCl-Lösung
- 0,1 M Natriumphosphatpuffer, pH = 7,4, in isotonischer NaCl-Lösung
- isotonischer NaCl-Lösung (4 mal)

gewaschen und 20 min bei 121 °C autoklaviert.

Beispiel 8

Ein poröses Trägermaterial wurde in der gleichen Weise wie in Beispiel 7 hergestellt, außer daß Schritt D vor Schritt C erfolgte.

Vergleichsbeispiele 4 und 5

Zum Vergleich wurden Adsorbentien wie in Beispiel 7 hergestellt, die jedoch sowohl auf der inneren als auch auf der äußeren Oberfläche ausschließlich mit Oberflächenmodifizierunaen $M_1$ (Vergleichsbeispiel 4) oder ausschließlich mit der Oberflächenmodifizierung $M_2$ (Vergleichsbeispiel 5) modifiziert waren.
Die Ergebnisse von Untersuchungen hinsichtlich der Bindungsfähigkeit für Thrombozyten bzw. Fibrinogen sind in Tabelle 3 zusammengefaßt.

**Tabelle 3**

| | Oberfläche des Trägermaterials | | Bindungsfähigkeit für | |
|---|---|---|---|---|
| | außen | innen | Thrombozyten | Fibrinogen |
| Bsp. 7 | $M_1$ | $M_2$ | nein | ja |
| Bsp. 8 | $M_1$ | $M_2$ | nein | ja |
| Vgl.-Bsp. 4 | $M_1$ | $M_1$ | nein | nein |
| Vgl.-Bsp. 5 | $M_2$ | $M_2$ | ja | ja |

Beispiel 9 (Herstellung eines porösen Trägermaterials durch Suspensionspolymerisation in Gegenwart eines wasserlöslichen Monomers in der wässrigen Phase)

1,5 g Polyvinylalkohol (W25/190, Erkol) und 744 g destilliertes Wasser wurden bis zur Auflösung - in einem Doppelmantelglasreaktor, der mit einem 4-Blatt-Flügelrührer ausgestattet war, gerührt. Anschließend wurde eine Lösung, bestehend aus 135 g Cyclohexanol, 13 g Dodecanol (FLUKA), 40 g Ethylenglykoldimethacrylat (RÖHM, Darmstadt), 60 g Glycidylmethacrylat (TRANSOL) und 1,1 g Azobisisobutyronitril (VAZO 64, DU PONT), zugegeben. Das Reaktionsgemisch wurde bei 270 Umdrehungen/min für 30 min gerührt und anschließend zum Starten der Polymerisation auf 65°C erwärmt. Bei Erreichen einer Temperatur von 60°C wurden dem Reaktionsgemisch 19 g Acrylsäure (Merck) zugegeben. Nach weiteren 5h bei 65°C und 4h bei 78°C, wurde für 1,5h auf 91°C erwärmt. Nach Abkühlen auf Raumtemperatur wurde das gebildete Perlpolymerisat abfiltriert, mehrfach mit Isopropanol gewaschen (12 mal mit 400ml) und fraktioniert.

100 g des vorstehend gebildeten feuchten Trägermaterials wurden mit 25 Gew.-%igem Ammoniak (FLUKA) für 3h bei Raumtemperatur auf einem Rotationsschüttler inkubiert. Anschließend wurde die Reaktionslösung mit 0,5 bar Überdruck vom Trägermaterial abfiltriert und dieses bis zur Neutralität mit destilliertem Wasser gewaschen.

Das vorstehend beschriebene Trägermaterial wird mit 4 mal 250 ml $NaH_2PO_4$-Puffer (pH = 6,8) (PP-Puffer) gewaschen. Anschließend wird das Trägermaterial für 2h bei 40°C mit 300 ml einer 0,4%igen Glutardialdehydlösung (FLUKA) in PP-Puffer auf dem Rotationsschüttler inkubiert, 12 mal mit jeweils 1l destilliertem Wasser gewaschen und 4 mal mit 1l PP-Puffer, auf pH = 6,8 äquilibriert. Danach wurden die Glutardialdehydgruppen mit 300ml einer 0;25 M Ethanolaminlösung (FLUKA) in destilliertem Wasser bei einem pH = 8 (4h, Raumtemperatur, Rotationsschüttler) umgesetzt und das gebildete Trägermaterial 4 mal mit je 250 ml PP-Puffer gewaschen. Das Trägermaterial wird dann mit 250 ml einer 5mM Ascorbinsäurelösung (FLUKA) in PP-Puffer für 4h bei Raumtemperatur auf dem Rotationsschüttler inkubiert (im Dunkeln) und mit jeweils 500 ml:

- 0,1 M Natriumacetatpuffer, pH = 4,6, in isotonischer NaCl-Lösung
- 0,1 M Natriumphosphatpuffer, pH = 9, in isotonischer NaCl-Lösung
- 0,1 M Natriumphosphatpuffer, pH = 7,4, in isotonischer NaCl-Lösung
- in isotonischer NaCl-Lösung (4 mal)

gewaschen und für 20 min bei 121 °C autoklaviert.

Vergleichsbeispiel 6

Zu Vergleichszwecken wurde ein poröses Trägermaterial wie in Beispiel 9 beschrieben hergestellt, außer daß keine Acrylsäure zu dem Reaktionsgemisch (d.h. zu der wässrigen Phase) gegeben wurde.

Vergleichsbeispiel 7

Zu Vergleichszwecken wurde ein Trägermaterial wie in Beispiel 9 hergestellt und mit Polyacrylsäure auf den inneren und äußeren Oberflächen des porösen Trägermaterials versehen.

Die Ergebnisse von Untersuchungen hinsichtlich der Bindungsfähigkeit der nach Beispiel 9 und Vergleichsbeispielen 6 und 7 hergestellten porösen Trägermaterialien für Thrombozyten bzw. Fibrinogen sind in Tabelle 4 zusammengefaßt.

**Tabelle 4**

| | Art der Oberflächenmodifizierung | Bindungsfähigkeit für | |
|---|---|---|---|
| | | Thrombozyten | Fibrinogen |
| Bsp. 9 | mit Polyacrylsäure an der äußeren Oberfläche der Beads | nein | ja |
| Vgl.- Bsp. 6 | ohne Polyacrylsäure an der äußeren Oberfläche der Beads | ja | ja |
| Vgl.- Bsp. 7 | Vergleichsmaterial mit Polyacrylsäure auf der inneren und äußeren Oberfläche der Beads | nein | nein |

**Patentansprüche**

1.  Verfahren zur Herstellung eines Adsorbens für Vollblut, umfassend die folgenden schritte in der angegebenen Reihenfolge :

    - Bereitstellen eines teilchenförmigen, porösen organischen Trägermaterials mit einer mittleren Porengröße von ≤ 1,5 $\mu$m, wobei maximal 50% des Porenvolumens in Poren vorliegen kann, welche eine Porengröße von > 1,5$\mu$m aufweisen, welches auf den äußeren und inneren Oberflächen modifizierbare Bindungsstellen aufweist,
    - Füllen der Poren des Trägermaterials mit mindestens einem, unter den Bedingungen des Füllens flüssigen Medium, welches unter den Bedingungen des Modifizierens mit einer Lösung nicht mischbar ist, mit welcher Bindungsstellen auf den Oberflächen des Trägermaterials vollständig modifiziert werden können,
    - vollständiges Modifizieren der Bindungsstellen auf den äußeren Oberflächen des Trägermaterials zu mindestens einer Oberflächenmodifizierung $M_1$, die nicht mit Blutzellen wechselwirkt,
    - gegebenenfalls Entfernen des Mediums, welches unter den Bedingungen des Modifizierens mit einer Lösung, mit welcher Bindungsstellen auf den äußeren Oberflächen des Trägermaterials vollständig modifiziert werden können, nicht mischbar ist, aus den Poren, und
    - Modifizieren der Bindungsstellen auf den inneren Oberflächen des Trägermaterials zu mindestens einer Oberflächenmodifizierung $M_2$ mit einer Lösung, welche zum Modifizieren der Oberflächenmodifizierung $M_1$ nicht befähigt ist, wobei die Oberflächenmodifizierung $M_2$ mit in Blut enthaltenen Substanzen wechselwirkt.

2.  Verfahren zur Herstellung eines Adsorbens für Vollblut, umfassend die folgenden Schritte in der angegebenen Reihenfolge :

    - Herstellen eines teilchenförmigen, porösen organischen Trägermaterials mit einer mittleren Porengröße von ≤1,5 $\mu$m, wobei maximal 50% des Porenvolumens in Poren vorliegen kann, welche eine Porengröße von > 1,5$\mu$m aufweisen, welches auf den äußeren und inneren Oberflächen modifizierbare Bindungsstellen aufweist, durch Suspensionspolymerisation, wobei eine hydrophobe Phase, umfassend mindestens ein Monomer, mindestens einen Vernetzer, ein porenbildendes Medium und einen Polymerisationsinitiator, in einer kontinuierlichen Phase, umfassend Wasser und mindestens ein Schutzkolloid, dispergiert wird, und die sich in der hydrophoben Phase befindlichen Monomere und Vernetzer polymerisiert werden, wobei die Poren des resultierenden teilchenförmigen porösen Trägermaterials mit dem porenbildenden Medium gefüllt sind,
    - vollständiges Modifizieren der modifizierbaren Bindungsstellen auf den äußeren Oberflächen der Trägermaterialteilchen zu mindestens einer Oberflächenmodifizierung $M_1$, die nicht mit Blutzellen wechselwirkt,
    - gegebenenfalls Entfernen des porenbildenden Mediums aus den Poren, und
    - Modifizieren der Bindungsstellen auf den inneren Oberflächen des Trägermaterials zu mindestens einer Oberflächenmodifizierung $M_2$ mit einer Lösung, welche zum Modifizieren der Oberflächenmodifizierung $M_1$ nicht befähigt ist, wobei die Oberflächenmodifizierung $M_2$ mit in Blut enthaltenen Substanzen wechselwirkt.

3.  Verfahren zur Herstellung eines Adsorbens für Vollblut, umfassend die folgenden Schritte in der angegebenen Reihenfolge :

    - Herstellen eines teilchenförmigen, porösen organischen Trägermaterials mit einer mittleren Porengröße von ≤1,5 $\mu$m, wobei maximal 50% des Porenvolumens in Poren vorliegen kann, welche eine Porengröße von > 1,5$\mu$m aufweisen, welches auf den äußeren und inneren Oberflächen modifizierbare Bindungsstellen aufweist, durch Suspensionspolymerisation, wobei eine hydrophobe Phase, umfassend mindestens ein Monomer, mindestens einen Vernetzer, ein porenbildendes Medium und einen Polymerisationsinitiator,

in einer kontinuierlichen Phase, umfassend Wasser, mindestens ein Schutzkolloid und mindestens ein wasserlösliches, in der hydrophoben Phase unlösliches Monomer, dispergiert wird, wobei die sich in der hydrophoben Phase befindlichen Monomere und Vernetzer unter Ausbildung eines porösen Kerns polymerisiert werden und das mindestens eine, wasserlösliche Monomer in der Phasengrenzfläche zwischen hydrophober und kontinuierlicher Phase zu mindestens einer Oberflächenmodifizierung $M_1$ polymerisiert, die nicht mit Blutzellen wechselwirkt, wobei mindestens ein Teil der sich aus dem wasserlöslichen Monomer bildenden Polymerketten kovalent an den sich bildenden porösen Kem gebunden wird,
- gegebenenfalls Entfernen des porenbildenden Mediums aus den Poren, und
- Modifizieren der Bindungsstellen auf den inneren Oberflächen des Trägermaterials zu mindestens einer Oberflächenmodifizierung $M_2$ mit einer Lösung, welche zum Modifizieren der Oberflächenmodifizierung $M_1$ nicht befähigt ist, wobei die Oberflächenmodifizierung $M_2$ mit in Blut enthaltenen Substanzen wechselwirkt.

4. Verfahren nach einem oder mehreren der Ansprüche 1 bis 3, wobei als die zum Modifizieren von Bindungsstellen auf den äußeren Oberflächen des Trägermaterials zu mindestens einer Oberflächenmodifizierung $M_1$ befähigte Lösung eine auf Wasser basierende Lösung verwendet wird.

5. Verfahren nach einem oder mehreren der Ansprüche 1 bis 4, wobei während einer Modifizierung $M_1$ der äußeren Oberflächen ein hydrophobes Medium als porenfüllendes bzw. porenbildendes Medium verwendet wird.

6. Verfahren nach Anspruch 5, wobei das hydrophobe Medium mindestens eines ist, welches aus der Gruppe, bestehend aus linearen und verzweigten, acyclischen oder cyclischen $C_1$- bis $C_{20}$-Alkanen, linearen und verzweigten, acyclischen oder cyclischen $C_5$- bis $C_{20}$-Alkanolen, linearen und verzweigten, acyclischen oder cyclischen $C_2$- bis $C_{30}$-Carbonsäureestern und aromatischen $C_6$- bis $C_{20}$-Kohlenwasserstoffen, ausgewählt ist.

7. Verfahren nach Anspruch 6, wobei das porenbildende Medium ein Gemisch aus mindestens zwei Komponenten, ausgewählt aus linearen, verzweigten oder cyclischen Alkoholen mit 5 bis 14 Kohlenstoffatomen und Estern von Mono- oder Dicarbonsäuren mit 2 bis 10 Kohlenstoffatomen, die mit ein- oder mehrwertigen Alkoholen mit 1 bis 6 Kohlenstoffatomen verestert sind, ist.

8. Verfahren zur Herstellung eines Adsorbens, umfassend die Schritte in der angegebenen Reihenfolge :

- Bereitstellen eines teilchenförmigen, porösen organischen Trägermaterials mit einer mittleren Porengröße von ≤1,5 $\mu$m, wobei maximal 50% des Porenvolumens in Poren vorliegen kann, welche eine Porengröße von > 1,5$\mu$m aufweisen, welches auf den äußeren Oberflächen und den inneren Oberflächen modifizierbare Bindungsstellen aufweist,
- vollständiges Modifizieren der Bindungsstellen auf den äußeren Oberflächen des Trägermaterials zu mindestens einer Oberflächenmodifizierung $M_1$, die nicht mit Blutzellen wechselwirkt, mittels einer Umsetzung, welche durch eine das Trägermaterial nicht durchdringende Strahlung bewirkt wird, und
- vollständiges Modifizieren der Bindungsstellen auf den inneren Oberflächen des Trägermaterials zu mindestens einer Oberflächenmodifizierung $M_2$, mittels einer Umsetzung, welche zum Modifizieren der Oberflächenmodifizierungen $M_1$ nicht befähigt ist, wobei die Oberflächenmodifizierung $M_2$ mit in Blut enthaltenen Substanzen wechselwirkt.

9. Verfahren nach Anspruch 2 oder 3, wobei das in der hydrophoben Phase enthaltene Monomer ein Epoxidgruppen enthaltendes Monomer oder ein Gemisch aus einem Epoxidgruppen enthaltenden Monomer und mindestens einem weiteren Monomer ist.

10. Verfahren nach Anspruch 9, wobei das Epoxidgruppen enthaltende Monomer ein Epoxidgruppen enthaltendes (Meth)Acrylsäuremonomer ist.

11. Verfahren nach Anspruch 10, wobei das Epoxidgruppen enthaltende (Meth)Acrylsäuremonomer Glycidyl(meth) acrylat ist.

12. Verfahren nach einem oder mehreren der Ansprüche 2, 3 und 9 bis 11, wobei der Vernetzer ein Monomer mit mindestens zwei polymerisierbaren Gruppen ist.

13. Verfahren nach Anspruch 12, wobei das Monomer mit mindestens zwei polymerisierbaren Gruppen ein Monomer auf Basis von (Meth)Acrylsäure ist.

**14.** Verfahren nach Anspruch 13, wobei das Monomer mit mindestens zwei polymerisierbaren Gruppen Ethylenglykoldi (meth)acrylat ist.

**15.** Verfahren nach einem oder mehreren der Ansprüche 2, 3 und 9 bis 14, wobei der Polymerisationsinitiator aus der Gruppe von Azoverbindungen, Peroxiden und Redoxinitiatoren ausgewählt ist.

**16.** Verfahren nach Anspruch 15, wobei der Polymerisationsinitiator Azobisisobutyronitril oder Dibenzoylperoxid ist.

**17.** Verfahren nach einem der Ansprüche 3 und 9 bis 16, wobei das wasserlösliche Monomer eine monofunktionale Vinylverbindung ist.

**18.** Verfahren nach einem der Ansprüche 3 und 9 bis 17, wobei das wasserlösliche Monomer eine oder mehrere polare Gruppe(n) aufweist.

**19.** Verfahren nach einem der Ansprüche 3 und 9 bis 18, wobei das wasserlösliche Monomer ausgewählt ist aus der Gruppe, umfassend Hydroxyalkylmethacrylate, Aminoalkylmethacrylate, N-Vinyl-2-pyrrolidon, Acryl- und Methacrylnitril, Acryl- und Methacrylsäure, Allylalkohol, Allylamin, 3-Allyloxy-1,2-propandiol, $(Ethylenglykol)_n$-vinylmethylether mit n=1 bis 10, N-Methylolacrylamid, 2-Acrylamido-2-methylpropansulfonsäure und Salze der Vinylbenzolsulfonsäure, Vinylsulfonsäure, sowie Kombinationen davon.

**20.** Verfahren nach einem der Ansprüche 3 und 9 bis 19, wobei der Anteil des wasserlöslichen Monomers 2 Gew.-% bis 80 Gew.-%, bezogen auf das Gesamtausgangsmonomergewicht des porösen Trägermaterials, beträgt.

**21.** Adsorbens für Vollblut in Form von sphärischen, nicht aggregierten Teilchen, welche ein organisches poröses Trägermaterial mit einer mittleren Porengröße von $\leq 1{,}5\ \mu m$ umfassen, wobei maximal 50 % des Porenvolumens in Poren vorliegen kann, welche eine Porengröße von $> 1{,}5\ \mu m$ aufweisen, wobei die äußeren Oberflächen des porösen Trägermaterials mindestens eine Oberflächenmodifizierung $M_1$ aufweisen, die nicht mit Blutzellen wechselwirkt, und die inneren Oberflächen des porösen Trägermaterials, d.h. die Oberflächen der Poren des porösen Trägermaterials, mindestens eine Oberflächenmodifizierung $M_2$ aufweisen, welche mit in Blut enthaltenen Substanzen wechselwirkt.

**22.** Adsorbens nach Anspruch 21, wobei die Teilchen des Adsorbens eine Komgröße von 50 bis 500 $\mu m$ aufweisen.

**23.** Adsorbens nach Anspruch 21 oder 22, wobei die innere Oberfläche des porösen Trägermaterials durch Aminierung oberflächenmodifiziert ist.

**24.** Adsorbens nach einem oder mehreren der Ansprüche 21 bis 23, wobei die äußere Oberfläche durch kovalente Bindung mindestens einer Gruppierung $M_1$ an Bindungsstellen auf der äußeren Oberfläche und/oder die innere Oberfläche durch kovalente Bindung mindestens einer Gruppierung $M_2$ an Bindungsstellen auf der inneren Oberfläche modifiziert ist/sind.

**25.** Adsorbens nach einem oder mehreren der Ansprüche 21 bis 24, wobei die mindestens eine Gruppierung $M_1$ min- destens eine Poly(carbonsäure), Albumin, Heparin, Heparansulfat und/oder Polymyxin ist.

**26.** Adsorbens nach einem oder mehreren der Ansprüche 21 bis 25, wobei die mindestens eine Gruppierung $M_1$ aus Polyacrylsäure und/oder Heparin ausgewählt ist.

**27.** Adsorbens nach einem oder mehreren der Ansprüche 21 bis 26, wobei die mindestens eine Gruppierung $M_2$ ein Immunadsorber ist.

**28.** Adsorbens nach einem oder mehreren der Ansprüche 21 bis 27, wobei die mindestens eine Gruppierung $M_2$ aus der Gruppe, bestehend aus dem Komplementfaktor C1q, Peptiden, Antikörpem wie monoklonalen oder polyklonalen Anti-Fibrinogen- oder Anti-Fibrin-Antikörpern und Antigenen wie synthetischen AIB-Blutgruppenantigenen ausgewählt ist.

**29.** Adsorbens nach einem oder mehreren der Ansprüche 21 bis 28, wobei das Trägermaterial ein von (Meth)Acrylsäureestem und/oder -amiden abgeleitetes Copolymer ist.

**30.** Adsorbens nach Anspruch 29, wobei das von (Meth)Acrylsäureestern und/oder -amiden abgeleitete Copolymer Epoxidgruppen aufweist.

**31.** Adsorbens nach Anspruch 29 oder 30, wobei das Copolymer durch Polymerisation von Ethylenglykoldi(meth)acrylat und Glycidyl(meth)acrylat und/oder Allylglycidylether hergestelltes, statistisches Copolymer ist.

**32.** Adsorbens nach Anspruch 29 oder 30, wobei das Copolymer ein durch Polymerisation der monomeren Einheiten

    (A) (Meth)Acrylamid in einer Menge von 10 bis 30 Gew.-%,
    (B) N,N'-Methylen-bis(meth)acrylamid in einer Menge von 30 bis 80 Gew.-%, und
    (C) Allylglycidylether und/oder Glycidyl(meth)acrylat in einer Menge von 10 bis 20 Gew.-%, jeweils bezogen auf das Gesamtgewicht der monomeren Einheiten,

hergestelltes, vernetztes statistisches Copolymer ist.

**33.** Adsorbens nach einem oder mehreren der Ansprüche 29 bis 32, wobei das Copolymer durch eine Suspensionspolymerisation hergestellt wurde.

**34.** Verwendung des Adsorbens nach einem oder mehreren der Ansprüche 21 bis 33 zum Abtrennen von Biomolekülen und/oder pathogenen Stoffen aus Vollblut.

**35.** Verwendung nach Anspruch 34, wobei die abzutrennenden Biomoleküle Immunglobuline, Fibrinogene und/oder Immunkomplexe sind.

**36.** Verwendung eines Adsorbens nach einem oder mehreren der Ansprüche 21 bis 33 zur Herstellung eines Adsorbers, umfassend ein Gehäuse und darin befindliches Adsorbens, zum Abtrennen von Biomolekülen und/oder pathogenen Stoffen aus Vollblut.

**37.** Verwendung nach Anspruch 36, wobei der Adsorber ein Volumen von 10 bis 1250 mi umfaßt.

**38.** Verwendung nach Anspruch 36 oder 37, wobei der Adsorber einen kopfseitigen Einlaßbereich und einen Auslaßbereich am Boden des Gehäuses aufweist.

**39.** Verwendung nach einem oder mehreren der Ansprüche 36 bis 38, wobei der Adsorber in seinem Auslaßbereich einen Filter aufweist.

**40.** Verwendung nach Anspruch 39, wobei der Filter ein Partikelfilter ist.

**41.** Verwendung nach einem oder mehreren der Ansprüche 36 bis 40, wobei sich am Boden des Gehäuses ein Sieb mit einer Maschenweite von mindestens 25 $\mu$m befindet.

**42.** Verwendung nach einem oder mehreren der Ansprüche 36 bis 41, wobei sich am Boden des Gehäuses ein Sieb mit einer Maschenweite von mindestens 40 $\mu$m befindet.

**Claims**

**1.** Method of producing an adsorbent for whole blood, comprising the following steps in the stated order:

    - providing a particulate porous organic carrier material having an average pore size of $\leq 1.5$ $\mu$m although up to 50% of the pore volume can be present in pores having a pore size of > 1.5 $\mu$m, the carrier material having modifiable binding sites on the outer and inner surfaces,
    - filling the pores of the carrier material with at least one medium which is liquid under the conditions of filling and which, under the conditions of modifying, is not miscible with a solution with which binding sites on the surfaces of the carrier material can be completely modified,
    - completely modifying the binding sites on the outer surfaces of the carrier material to yield at least one surface modification $M_1$ which does not interact with blood cells,
    - optionally removing from the pores the medium which, under the conditions of the modifying, is not miscible with a solution with which binding sites on the outer surfaces of the carrier material can be completely modified, and

- modifying the binding sites on the inner surfaces of the carrier material to yield at least one surface modification $M_2$ with a solution not effective for modifying the surface modification $M_1$, the surface modification $M_2$ interacting with substances present in blood.

2. Method of producing an adsorbent for whole blood, comprising the following steps in the stated order:

- producing a particulate porous organic carrier material having an average pore size of $\leq 1.5\ \mu$m although up to 50% of the pore volume can be present in pores having a pore size of > 1.5 $\mu$m, the carrier material having modifiable binding sites on the outer and inner surfaces, by suspension polymerization comprising a hydrophobic phase comprising at least one monomer, at least one crosslinker, a pore-forming medium and a polymerization initiator

being dispersed in a continuous phase comprising water and at least one protective colloid and the monomers and crosslinkers present in the hydrophobic phase being polymerized, the pores of the resulting particulate porous carrier material having been filled with the pore-forming medium,

- completely modifying the modifiable binding sites on the outer surfaces of the carrier material particles to yield at least one surface modification $M_1$ which does not interact with blood cells,
- optionally removing the pore-forming medium from the pores, and
- modifying the binding sites on the inner surfaces of the carrier material to yield at least one surface modification $M_2$ with a solution not effective for modifying the surface modification $M_1$, the surface modification $M_2$ interacting with substances present in blood.

3. Method of producing an adsorbent for whole blood, comprising the following steps in the stated order:

- producing a particulate porous organic carrier material having an average pore size of $\leq 1.5\ \mu$m although up to 50% of the pore volume can be present in pores having a pore size of > 1.5 $\mu$m, the carrier material having modifiable binding sites on the outer and inner surfaces, by suspension polymerization comprising a hydrophobic phase comprising at least one monomer, at least one crosslinker, a pore-forming medium and a polymerization initiator

being dispersed in a continuous phase comprising water and at least one protective colloid and at least one water-soluble monomer insoluble in the hydrophobic phase, the monomers and crosslinkers present in the hydrophobic phase being polymerized to form a porous core and the at least one water-soluble monomer in the phase interface between the hydrophobic phase and the continuous phase being polymerized to yield at least one surface modification $M_1$ which does not interact with blood cells, at least a portion of the polymer chains forming from the water-soluble monomer becoming covalently bonded to the resulting porous core,

- optionally removing the pore-forming medium from the pores, and
- modifying the binding sites on the inner surfaces of the carrier material to yield at least one surface modification $M_2$ with a solution not effective for modifying the surface modification $M_1$, the surface modification $M_2$ interacting with substances present in blood.

4. Method according to one or more of Claims 1 to 3, wherein the solution effective for modifying binding sites on the outer surfaces of the carrier material to yield at least one surface modification $M_1$ is a waterborne solution.

5. Method according to one or more of Claims 1 to 4, wherein a hydrophobic medium is used as pore-filling or pore-forming medium during a modification $M_1$ of the outer surfaces.

6. Method according to Claim 5, wherein the hydrophobic medium is at least one selected from the group consisting of linear and branched, acyclic or cyclic $C_1$- to $C_{20}$-alkanes, linear and branched, acyclic or cyclic $C_5$- to $C_{20}$-alkanols, linear and branched, acyclic or cyclic $C_2$- to $C_{30}$-carboxylic esters and aromatic $C_6$- to $C_{20}$-hydrocarbons.

7. Method according to Claim 6, wherein the pore-forming medium is a mixture of at least two components selected from linear, branched or cyclic alcohols having 5 to 14 carbon atoms and esters of mono- or dicarboxylic acids having 2 to 10 carbon atoms esterified with mono- or polyhydric alcohols having 1 to 6 carbon atoms.

8. Method of producing an adsorbent, comprising the steps in the stated order:

- providing a particulate porous organic carrier material having an average pore size of $\leq 1.5\ \mu$m although up

to 50% of the pore volume can be present in pores having a pore size of > 1.5 $\mu$m, the carrier material having modifiable binding sites on the outer and inner surfaces,

- completely modifying the binding sites on the outer surfaces of the carrier material to yield at least one surface modification $M_1$ which does not interact with blood cells by means of a reaction due to radiation which does not pass through the carrier material, and
- completely modifying the binding sites on the inner surfaces of the carrier material to yield at least one surface modification $M_2$ by means of a reaction not effective for modifying the surface modifications $M_1$, the surface modification $M_2$ interacting with substances present in blood.

9. Method according to Claim 2 or 3, wherein the monomer present in the hydrophobic phase is an epoxy-containing monomer or a mixture of an epoxy-containing monomer and at least one further monomer.

10. Method according to Claim 9, wherein the epoxy-containing monomer is an epoxy-containing (meth)acrylic acid monomer.

11. Method according to Claim 10, wherein the epoxy-containing methacrylic acid monomer is glycidyl (meth)acrylate.

12. Method according to one or more of Claims 2, 3 and 9 to 11, wherein the crosslinker is a monomer having at least two polymerizable groups.

13. Method according to Claim 12, wherein the monomer having at least two polymerizable groups is a monomer based on (meth)acrylic acid.

14. Method according to Claim 13, wherein the monomer having at least two polymerizable groups is ethylene glycol di(meth)acrylate.

15. Method according to one or more of Claims 2, 3 and 9 to 14, wherein the polymerization initiator is selected from the group of azo compounds, peroxides and redox initiators.

16. Method according to Claim 15, wherein the polymerization initiator is azobisisobutyronitrile or dibenzoyl peroxide.

17. Method according to any one of Claims 3 and 9 to 16, wherein the water-soluble monomer is a monofunctional vinyl compound.

18. Method according to one or more of Claims 3 and 9 to 17, wherein the water-soluble monomer has one or more polar group(s).

19. Method according to one or more of Claims 3 and 9 to 18, wherein the water-soluble monomer is selected from the group comprising hydroxyalkyl methacrylates, aminoalkyl methacrylates, N-vinyl-2-pyrrolidone, acrylonitrile, methacrylonitrile, acrylic acid, methacrylic acid, allyl alcohol, allylamine, 3-allyloxy-1,2-propanediol, (ethylene glycol)$_n$ vinyl methyl ether where n = 1 to 10, N-methylolacrylamide, 2-acrylamido-2-methylpropanesulfonic acid and salts of vinylbenzenesulphonic acid, vinylsulphonic acid and also combinations thereof.

20. Method according to any one of Claims 3 and 9 to 19, wherein the fraction of water-soluble monomer is in the range from 2% by weight to 80% by weight, based on the total starting monomer weight of the porous carrier material.

21. Adsorbent for whole blood in the form of spherical non-aggregated particles comprising an organic porous carrier material having an average pore size of ≤ 1.5 $\mu$m although up to 50% of the pore volume can be present in pores having a pore size of > 1.5 $\mu$m, the outer surfaces of the porous carrier material comprising at least one surface modification $M_1$ which does not interact with blood cells, and the inner surfaces of the porous carrier material, i.e. the surfaces of the pores of the porous carrier material, comprising at least one surface modification $M_2$ which interacts with substances present in blood.

22. Adsorbent according to Claim 21, wherein the particles of the adsorbent have a particle size in the range from 50 to 500 $\mu$m.

23. Adsorbent according to Claim 21 or 22, wherein the inner surface of the porous carrier material is surface modified by amination.

24. Adsorbent according to one or more of Claims 21 to 23, wherein the outer surface is modified by covalent bonding of at least one grouping $M_1$ to binding sites on the outer surface and/or the inner surface is modified by covalent bonding of at least one grouping $M_2$ to binding sites on the inner surface.

25. Adsorbent according to one or more of Claims 21 to 24, wherein the at least one grouping $M_1$ is at least one poly (carboxylic acid), albumin, heparin, heparan sulphate and/or polymyxin.

26. Adsorbent according to one or more of Claims 21 to 25, wherein the at least one grouping $M_1$ is selected from polyacrylic acid and/or heparin.

27. Adsorbent according to one or more of Claims 21 to 26, wherein the at least one grouping $M_2$ is an immunoadsorber.

28. Adsorbent according to one or more of Claims 21 to 27, wherein the at least one grouping $M_2$ is selected from the group consisting of the complement factor C1q, peptides, antibodies such as monoclonal or polyclonal anti-fibrinogen or anti-fibrin antibodies and antigens such as synthetic A/B blood group antigens.

29. Adsorbent according to one or more of Claims 21 to 28, wherein the carrier material is a copolymer derived from (meth)acrylic esters and/or (meth)acrylamides.

30. Adsorbent according to Claim 29, wherein the copolymer derived from (meth)acrylic esters and/or (meth)acrylamides comprises epoxy groups.

31. Adsorbent according to Claim 29 or 30, wherein the copolymer is random copolymer produced by polymerization of ethylene glycol di(meth)acrylate and glycidyl (meth)acrylate and/or allyl glycidyl ether.

32. Adsorbent according to Claim 29 or 30, wherein the copolymer is a crosslinked random copolymer produced by polymerization of the monomeric units

(A) (meth)acrylamide in an amount of 10% to 30% by weight,
(B) N,N'-methylenebis(meth)acrylamide in an amount of 30 to 80% by weight, and
(C) allyl glycidyl ether and/or glycidyl (meth)acrylate in an amount of 10% to 20% by weight, all based on the total weight of the monomeric units.

33. Adsorbent according to one or more of Claims 29 to 32, wherein the copolymer was produced by suspension polymerization.

34. Use of the absorbent according to one or more of Claims 21 to 33 for separating biomolecules and/or pathogenic entities from whole blood.

35. Use according to Claim 34, wherein the biomolecules to be separated off are immunoglobulins, fibrinogens and/or immunocomplexes.

36. Use of an adsorbent according to one or more of Claims 21 to 33 for producing an adsorber comprising a housing and an adsorbent present therein, for separating biomolecules and/or pathogenic entities from whole blood.

37. Use according to Claim 36, wherein the adsorber has a volume of 10 to 1250 ml.

38. Use according to Claim 36 or 37, wherein the adsorber comprises a head-side inlet region and an outlet region at the bottom of the housing.

39. Use according to one or more of Claims 36 to 38, wherein the adsorber has a filter in its outlet region.

40. Use according to Claim 39, wherein the filter is a particle filter.

41. Use according to one or more of Claims 36 to 40, wherein a sieve having a mesh size of at least 25 $\mu$m is situated at the bottom of the housing.

42. Use according to one or more of Claims 36 to 41, wherein a sieve having a mesh size of at least 40 $\mu$m is situated

at the bottom of the housing.

**Revendications**

1. Procédé de production d'un adsorbant pour le sang complet, comprenant les étapes suivantes, dans l'ordre indiqué :

- la mise à disposition d'un matériau de support organique poreux sous forme de particules, présentant une taille de pores moyenne $\leq 1,5$ $\mu$m, sachant qu'au maximum 50 % du volume des pores peuvent être constitués par les pores présentant une taille de pores > 1,5 $\mu$m qui présente sur ses surfaces externe et interne des sites de liaison modifiables,
- le remplissage des pores du matériau de support avec au moins un milieu liquide dans les conditions du remplissage qui, dans les conditions de modification, n'est pas miscible avec une solution avec laquelle des sites de liaison peuvent être complètement modifiés sur les surfaces du matériau de support,
- une modification complète des sites de liaison sur la surface externe du matériau de support jusqu'à une modification de surface $M_1$ qui n'interagit pas avec les cellules sanguines,
- l'élimination éventuelles du milieu se trouvant dans les pores qui, dans les conditions de modification avec une solution avec laquelle des sites de liaison sur la surface externe du matériau de support, peuvent être complètement modifiés, n'est pas miscible, et
- la modification des sites de liaison sur les surfaces internes du matériau de support jusqu'à au moins une modification de surface $M_2$ avec une solution qui peut modifier la modification de surface $M_1$, la modification de surface $M_2$ pouvant interagir avec les substances contenues dans le sang.

2. Procédé de production d'un adsorbant pour le sang complet, comprenant les étapes suivantes, dans l'ordre indiqué :

- la production d'un matériau de support organique poreux sous forme de particules, présentant une taille de pores moyenne $\leq 1,5$ $\mu$m, sachant qu'au maximum 50 % du volume des pores peuvent être constitués par les pores présentant une taille de pores > 1,5 $\mu$m qui présente sur ses surfaces externe et interne des sites de liaison modifiables, par polymérisation en suspension, dans laquelle une phase hydrophobe comprenant au moins un monomère, au moins un agent de réticulation, un milieu de formation de pores et un initiateur de polymérisation, est dispersée dans une phase continue comprenant de l'eau et au moins un colloïde protecteur et les monomères et agents de réticulations se trouvant dans la phase hydrophobe sont polymérisés, moyennant quoi les pores du matériau de support poreux sous forme de particules obtenus sont remplis par le milieu formant des pores,
- la modification complète des sites de liaison modifiables sur la surface externe des particules du matériau de support jusqu'à au moins une modification de surface $M_1$ qui n'interagit pas avec les cellules sanguines,
- l'élimination éventuelles du milieu formant les pores, se trouvant dans les pores, et
- la modification des sites de liaison sur les surfaces internes du matériau de support jusqu'à au moins une modification de surface $M_2$ avec une solution qui ne peut pas modifier la modification de surface $M_1$, la modification de surface $M_2$ pouvant interagir avec les substances contenues dans le sang.

3. Procédé de production d'un adsorbant pour le sang complet, comprenant les étapes suivantes, dans l'ordre indiqué

- production d'un matériau de support organique poreux sous forme de particules, présentant une taille de pores moyenne $\leq 1,5$ $\mu$m, sachant qu'au maximum 50 % du volume des pores peuvent être constitués par les pores présentant une taille de pores > 1,5 $\mu$m qui présente sur ses surfaces externe et interne des sites de liaison modifiables, par polymérisation en suspension, dans laquelle une phase hydrophobe comprenant au moins un monomère, au moins un agent de réticulation, un milieu de formation de pores et un initiateur de polymérisation, est dispersée dans une phase continue comprenant de l'eau et au moins un colloïde protecteur et au moins un monomère hydrosoluble, insoluble dans la phase hydrophobe, moyennant quoi les monomères et agents de réticulation se trouvant dans la phase hydrophobe sont polymérisés en formant un noyau poreux et au moins un monomère hydrosoluble est polymérisé dans la zone limite des phases entre la phase hydrophobe et la phase continue, jusqu'à au moins une modification de surface $M_1$ qui n'interagit pas avec les cellules sanguines, sachant qu'au moins une partie des chaînes de polymère se formant à partir du monomère hydrosoluble est liée de manière covalente au noyau poreux formé,
- l'élimination éventuelle du milieu formant les pores, se trouvant dans les pores, et
- la modification des sites de liaison sur les surfaces internes du matériau de support jusqu'à au moins une modification de surface $M_2$ avec une solution qui ne peut pas modifier la modification de surface $M_1$, la modi-

fication de surface $M_2$ pouvant interagir avec les substances contenues dans le sang.

4. Procédé selon une ou plusieurs des revendications 1 à 3, dans lequel on utilise comme solution pouvant modifier les sites de liaison sur les surfaces externes du matériau de support jusqu'à au moins une modification de surface $M_1$, une solution à base d'eau.

5. Procédé selon une ou plusieurs des revendications 1 à 4, dans lequel on utilise pendant une modification $M_1$ de la surface externe, un milieu hydrophobe comme milieu de remplissage ou de formation de pores

6. Procédé selon la revendication 5, dans lequel le milieu hydrophobe est au moins un milieu qui est choisi dans le groupe comprenant des alcanes en $C_1$ à $C_{20}$ linéaires ou ramifiés, acycliques ou cycliques, des alcanols en $C_5$ à $C_{20}$, linéaires ou ramifiés, acycliques ou cycliques, des esters d'acide carboxylique linéaires ou ramifiés, acycliques ou cycliques et des hydrocarbures aromatiques en $C_6$ à $C_{20}$.

7. Procédé selon la revendication 6, dans lequel le milieu formant les pores est un mélange constitué d'au moins deux composants choisis parmi les alcools linéaires, ramifiés ou cycliques comportant 5 à 14 atomes de carbone et des esters d'acides mono- ou dicarboxyliques comportant 2 à 10 atomes de carbone qui sont estérifiés par des alcools mono- ou multivalents comportant 1 à 6 atomes de carbone.

8. Procédé de production d'un adsorbant, comprenant les étapes suivantes dans l'ordre indiqué :

   - la mise à disposition d'un matériau de support organique poreux sous forme de particules, présentant une taille de pores moyenne $\leq 1,5\ \mu$m, sachant qu'au maximum 50 % du volume des pores peuvent être constitués par les pores présentant une taille de pores $> 1,5\ \mu$m qui présente sur ses surfaces externe et interne des sites de liaison modifiables,
   - la modification complète des sites de liaison sur la surface externe du matériau de support jusqu'à au moins une modification de surface $M_1$ qui n'interagit pas avec les cellules sanguines, par une réaction qui est produite par un rayonnement ne pénétrant pas dans le matériau de support, et
   - la modification complète des sites de liaison sur la surface interne du matériau de support jusqu'à au moins une modification de surface $M_2$ par une réaction qui ne peut pas modifier les modifications de surface $M_1$, la modification de surface $M_2$ interagissant avec les substances contenues dans le sang.

9. Procédé selon la revendication 2 ou 3, dans lequel le monomère contenu dans la phase hydrophobe est un monomère contenant des groupes époxydes ou un mélange d'un monomère contenant des groupes époxydes et au moins d'un autre monomère.

10. Procédé selon la revendication 9, dans lequel le monomère contenant des groupes époxydes est un monomère d'acide (méth)acrylique contenant des groupes époxydes.

11. Procédé selon la revendication 10, dans lequel le monomère d'acide (méth)acrylique contenant des groupes époxydes est le glycidyl(méth)acrylate.

12. Procédé selon une ou plusieurs des revendications 2, 3 et 9 à 11, dans lequel l'agent de réticulation est un monomère comportant au moins deux groupes polymérisables.

13. Procédé selon la revendication 12, dans lequel le monomère comportant au moins deux groupes polymérisables est un monomère à base d'acide (méth)acrylique.

14. Procédé selon la revendication 13, dans lequel le monomère comportant au moins deux groupes polymérisables est l'éthylène-glycol-di(méth)acrylate.

15. Procédé selon l'une ou plusieurs des revendications 2, 3 et 9 à 14, dans lequel l'initiateur de polymérisation est choisi dans le groupe des composés azo, des peroxydes et des initiateurs redox.

16. Procédé selon la revendication 15, dans lequel l'initiateur de polymérisation est l'azobis-isobutyronitrile ou le dibenzoylperoxyde.

17. Procédé selon l'une quelconque des revendications 3 et 9 à 16, dans lequel le monomère hydrosoluble est un composé vinylique monofonctionnel.

18. Procédé selon l'une quelconque des revendications 3 et 9 à 17, dans lequel le monomère hydrosoluble présente un ou plusieurs groupe(s) polaire (s) .

19. Procédé selon l'une quelconque des revendications 3 et 9 à 18, comprenant l'hydroxyalkylméthacrylate, l'aminoalkylméthacrylate, la N-vinyl-2-pyrrolidone, l'acryl- et méthacrylnitrile, l'acide acrylique et méthacrylique, l'alcool d'allyle, l'allylamine, le 3-allyloxy-1,2-propane-diol, l'(éthylèneglycol)$_n$-vinylméthyléther dans lequel n = 1 à 10, le N-méthylolacrylamide, l'acide 2-acrylamido-2-méthylpropane-sulfonique et les sels d'acide vinylbenzolsulfonique, d'acide vinylsulfonique et leurs combinaisons.

20. Procédé selon l'une quelconque des revendications 3 et 9 à 19, dans lequel la proportion de monomère hydrosoluble est de 2 % en poids à 80 % en poids par rapport au poids total du monomère de départ du matériau de support poreux.

21. Adsorbant pour le sang complet, sous la forme de particules sphériques non agrégées qui comprennent un matériau de support organique poreux présentant une taille de pores moyenne ≤ 1,5 $\mu$m, sachant qu'au maximum 50 % du volume des pores peuvent être constitués par les pores présentant une taille de pores > 1,5 $\mu$m, dans lequel les surfaces externes du matériau de support poreux présentent au moins une modification de surface $M_1$ qui n'interagit pas avec les cellules sanguines, et les surfaces internes du matériau de support poreux, c'est-à-dire les surfaces des pores du matériau de support poreux présentent au moins une modification de surface $M_2$ qui interagit avec les substances contenues dans le sang.

22. Adsorbant selon la revendication 21, dans lequel les particules d'adsorbant présentent une taille de particules de 50 à 500 $\mu$m.

23. Adsorbant selon la revendication 21 ou 22, dans lequel la surface interne du matériau de support poreux est modifiée en surface par amination.

24. Adsorbant selon l'une ou plusieurs des revendications 21 à 23, dans lequel la surface externe est modifiée par liaison covalente d'au moins un groupe $M_1$ aux sites de liaison présents sur la surface externe et/ou la surface interne est modifiée par liaison covalente d'au moins un groupe $M_2$ aux sites de liaison présents sur la surface interne.

25. Adsorbant selon l'une ou plusieurs des revendications 21 à 24, dans lequel au moins un groupe $M_1$ est au moins un acide (poly)carboxylique, l'albumine, l'héparine, le sulfate d'héparane et/ou la polymyxine.

26. Adsorbant selon l'une ou plusieurs des revendications 21 à 25, dans lequel au moins un groupe $M_1$ est choisi parmi l'acide polyacrylique et/ou l'héparine.

27. Adsorbant selon l'une ou plusieurs des revendications 21 à 26, dans lequel au moins le groupe $M_2$ est un adsorbant immunologique.

28. Adsorbant selon l'une ou plusieurs des revendications 21 à 27, dans lequel au moins un groupe $M_2$ est choisi dans le groupe constitué du facteur complémentaire C1q, de peptides, d'anticorps tels que les anticorps monoclonaux ou polyclonaux anti-fibrinogène ou anti-fibrine et des antigènes tels que les antigènes synthétiques des groupes sanguins A/B.

29. Adsorbant selon l'une ou plusieurs des revendications 21 à 28, dans lequel le matériau de support est un copolymère dérivé d'esters et/ou d'amides d'acide (méth)acrylique.

30. Adsorbant selon la revendication 29, dans lequel le copolymère dérivé d'esters et/ou d'amides (méth)acryliques présente des groupes époxydes.

31. Adsorbant selon la revendication 29 ou 30, dans lequel le copolymère est un copolymère statique produit par polymérisation d'éthylèneglycol-di(méth)acrylate et de glycidyl(méth)acrylate et/ou d'allylglycidyléther.

32. Adsorbant selon la revendication 29 ou 30, dans lequel le copolymère est un copolymère statique réticulé produit par polymérisation des motifs monomériques

(A) (méth) acrylamide en une quantité de 10 à 30 % en poids,

(B) N,N'-méthylène-bis-(méth)acrylamide en une quantité de 30 à 80 % en poids, et

(C) allylglycidyléther et/ou glycidyl(méth)-acrylate en une quantité de 10 à 20 % en poids, respectivement par rapport au poids total des motifs monomériques.

33. Adsorbant selon l'une ou plusieurs des revendications 29 à 32, dans lequel le copolymère est produit par polymérisation en suspension.

34. Utilisation de l'adsorbant selon l'une ou plusieurs des revendications 21 à 33, pour séparer des biomolécules et/ou des substances pathogènes dans le sang complet.

35. Utilisation selon la revendication 34, dans laquelle les biomolécules à séparer sont les immunoglobulines, les fibrinogènes et/ou des complexes immunologiques.

36. Utilisation d'un adsorbant selon l'une ou plusieurs des revendications 21 à 33 pour la production d'un adsorbant, comprenant un réceptacle et un adsorbant se trouvant dans celui-ci, pour séparer des biomolécules et/ou des substances pathogènes du sang complet.

37. Utilisation selon la revendication 36, dans laquelle l'adsorbant comprend un volume de 10 à 1250 ml.

38. Utilisation selon la revendication 36 ou 37, dans laquelle l'adsorbant présente une zone d'entrée du haut et une zone de sortie du fond du réceptacle.

39. Utilisation selon l'une ou plusieurs des revendications 36 à 38, dans laquelle l'adsorbant présente un filtre dans sa zone de sortie.

40. Utilisation selon la revendication 39, dans laquelle le filtre est un filtre à particules.

41. Utilisation selon l'une ou plusieurs des revendications 36 à 40, dans laquelle se trouve au fond du réceptacle, un tamis présentant une largeur de mailles d'au moins 25 $\mu$m.

42. Utilisation selon l'une ou plusieurs des revendications 36 à 41, dans laquelle se trouve au fond du réceptacle, un tamis présentant une largeur de mailles d'au moins 40 $\mu$m

**Fig. 1**

Fig. 2

Fig. 3